# FASCICULE DE BREVET EUROPEEN

(11) **EP 1 497 467 B1**
(45) Date de publication et mention de la délivrance du brevet: **19.08.2009**
(21) Numéro de dépôt: 03746850.1
(22) Date de dépôt: 22.04.2003
(51) Int. Cl.: C12Q 1/68, C12Q 1/70, A61K 31/7088, A61K 38/02, A61K 39/21, C07K 14/15, C07K 16/10, C12N 15/48

(54) **OLIGONUCLEOTIDE ISSUS DES SEQUENCES CODANT POUR LA COMPOSANTE DE SURFACE DES PROTEINES D'ENVELOPPE DES PTLV ET LEURS UTILISATIONS**
OLIGONUKLEOTIDE VON FÜR DIE OBERFLÄCHENKOMPONENTE DES PTLV-HÜLLPROTEINS CODIERENDEN SEQUENZEN UND VERWENDUNGEN DAVON
OLIGONUCLEOTIDES FROM SEQUENCES CODING FOR THE SURFACE COMPONENT OF PTLV ENVELOPE PROTEINS AND USES THEREOF

(30) Priorité: 22.04.2002 FR 0205001
(43) Date de publication de la demande: 19.01.2005
(73) Titulaire: CENTRE NATIONAL DE LA RECHERCHE SCIENTIFIQUE (CNRS), 75794 Paris Cedex 16 (FR); Universite de Montpellier II, 34095 Montpellier Cedex 5 (FR)
(72) Inventeur: KIM, Félix Jinhyun, NYC, NY 10044 (US); MANEL, Nicolas Gabriel Albert, F-34080 Montpellier (FR); SITBON, Marc Khamous Michel, F-34000 Montpellier (FR)
(74) Mandataire: Grosset-Fournier, Chantal Catherine
(86) Numéro de dépôt international: PCT/FR2003/001274
(87) Numéro de publication internationale: WO 2003/088979

(56) Documents cités:
- EP-A- 0 384 566
- WO-A-00/46403
- SUAREZ DAVID L ET AL: "Identification of hypervariable and conserved regions in the surface envelope gene in the bovine lentivirus." VIROLOGY, vol. 212, no. 2, 1995, pages 728-733, XP002253831 ISSN: 0042-6822
- RAMIREZ E. ET AL.: "Genetic characterization and phylogeny of human T-cell lymphotropic virus type I from Chile." VIRUS RESEARCH, vol. 84, 20 March 2002 (2002-03-20), XP001148390
- DUBE S. ET AL.: "Degenerate and specific PCR assays for the detection of bovine leukaemia virus and primate T cell leukaemia/lymphoma virus pol DNA and RNA: phylogenetic comparisons of amplified sequences from cattle and primates from around the world." JOURNAL OF GENERAL VIROLOGY, vol. 78, 1997, pages 1389-1398, XP002233752
- GRAY G. S. ET AL.: "Envelope gene sequence of HTLV-1 isolate MT-2 and its comparison with other HTLV-1 isolates." VIROLOGY, vol. 177, 1990, pages 391-395, XP008014557
- CALATTINI ET AL: "Discovery of a new human T-cell lymphotrophic virus (HTLV-3) in Central Africa" RETROVIROLOGY, vol. 2, 9 mai 2005 (2005-05-09), page 30,
- WOLFE ET AL: "Emergence of unique primate T-lymphotrophic viruses among central African bushmeat hunters" PNAS, vol. 102, no. 22, 31 mai 2005 (2005-05-31), pages 7994-7999,

## Description

La présente invention a pour objet des oligonucléotides issus des séquences nucléotidiques codant pour la région aminoterminale de la composante de surface des protéines d'enveloppe des virus des lymphomes/leucémies T chez les primates, regroupés sous la désignation PTLV, et leurs utilisations dans le cadre de la détection de toute souche de PTLV ou de souches virales apparentées.

La présente invention découle de l'identification par les Inventeurs de motifs peptidiques de la SU qui conviennent à la synthèse d'oligonucléotides pouvant être utilisés pour la détection et l'amplification de séquences pan-PTLV comprenant ces motifs. Les inventeurs ont mis au point une méthode permettant l'amplification de telles séquences, leur clonage et séquençage. La présente invention permet notamment la détection de séquences individuelles présentes dans un mélange de séquences des différents types. L'optimisation pour certains des motifs peptidiques ainsi identifiés a déjà permis la caractérisation de variants PTLV jamais encore décrits, ainsi que de détecter des séquences PTLV dont la présence dans les échantillons testés n'était pas suspectée. L'application généralisée de la présente invention permettra la détection et la caractérisation soit de nouvelles séquences apparentées aux SU de PTLV, soit de séquences déjà connues dans de nouveaux contextes pathologiques ou non.

La recherche de séquences des rétrovirus humains ou de primates est primordiale dans de nombreux contextes. De manière non-exhaustive, ces recherches intéressent le criblage de matériels biologiques (produits dérivés du sang, par exemple), le diagnostic (recherche de l'étiologie de syndromes multiples couvrant leucémies, maladies dégénératives, maladies autoimmunes, etc), les études épidémiologiques et anthropologiques des différents groupes humains, le séquençage des génomes (composition et marqueurs rétroviraux polymorphiques des génomes), le criblage de nouveaux médicaments (définition de nouvelles cibles), etc.

Dans le cas des PTLV, nous relèverons deux exemples des problèmes associés à la détection de leurs séquences. Dans le premier exemple, des individus, généralement réunis sous le terme de " séroindéterminés ", présentent une réponse immune anti-HTLV dite "incomplète ", dirigée contre certains antigènes seulement des PTLV, alors qu'aucune séquence correspondant à des PTLV ne peut être amplifiée à partir d'échantillons sanguins de ces patients. Dans les cas les mieux documentés la recherche de telles séquences se fait sur des régions conservées des gènes *gag, pol, env* et *tax*. DUBE S. et al. ("Degenerate and specific PCR assays for the detection of bovine leukaemia virus and primate T cell leukaemia/lymphoma virus pol DNA and RNA: phylogenetic comparisons of amplified sequences from cattle and primates from around the world." JOURNAL OF GENERAL VIROLOGY, vol. 78, 1997, pages 1389-1398) décrit des oligonucléotides dégénérés (p. 1391, § "Design of primer and probe oligonucleotides") ainsi que leur utilisation pour la détection de souches de PTLV ou virus apparentés (BLV, virus de la leucémie bovine) par l'amplification de séquences pol codant la polymerase virale desdits virus. Dans le cas du gène d'enveloppe, la partie amino terminale de la SU est écartée de cette approche du fait de sa variabilité. La région amino terminale, de la composante de surface (SU) des enveloppes des rétrovirus de primates humains et non-humains de type HTLV et STLV (regroupés ici sous le terme PTLV) est notamment responsable de la reconnaissance du ou des récepteurs cellulaires pour l'enveloppe (Kim et coll, 2000). À ce jour, aucune méthode d'amplification dans cette région directement applicable aux trois types de PTLV (application dite pan-PTLV) n'a été décrite.
Ainsi, généralement seule est considérée l'amplification de motifs présents dans les parties les plus conservées de la composante transmembranaire de l'enveloppe (TM). Toutefois, dans la mesure où la variabilité de la SU est un élément essentiel de la biologie adaptative des rétrovirus, la mise au point d'une approche basée sur sa détection représente un objectif particulièrement intéressant.

Un objet décrit consiste en l'utilisation de couples d'oligonucléotides dégénérés en orientation 5' et 3' issus des séquences nucléotidiques codant pour la région aminoterminale de la composante de surface (SU) des protéines d'enveloppe des virus des lymphomes / leucémies T chez les primates, regroupés sous la désignation PTLV, ces virus étant encore désignés HTLV chez l'homme et STLV chez le singe, à savoir de la région correspondant aux fragments protéiques délimités du côté N-terminal par un acide aminé situé entre les positions 75 à 90, et du côté C-terminal par un acide aminé situé entre les positions 230 à 245 des protéines d'enveloppe des différentes souches des PTLV, ou de virus portant des séquences apparentées aux SU des PTLV,
pour la mise en oeuvre de procédés de détection de toute souche de PTLV, à savoir de toute souche appartenant aux HTLV-1, HTLV-2, STLV-1, STLV-2, et STLV-3, ainsi que de toute souche de virus apparentés aux PTLV, à savoir de toute souche dont la séquence en acides aminés déduite de la séquence nucléotidique codant pour la région aminoterminale de la SU présente un taux d'homologie d'au moins environ 30% avec les séquences en acides aminés codées par les séquences nucléotidiques correspondantes chez les PTLV, notamment pour la détection de nouveaux variants des PTLV, ou de virus, nouveaux ou non, comportant des séquences apparentées aux SU des PTLV, le cas échéant dans de nouveaux contextes pathologiques, lesdits procédés comprenant une étape d'amplification, à partir d'un échantillon biologique susceptible de contenir des PTLV, et à l'aide des oligonucléotides 5' et 3' dégénérés susmentionnés utilisés en tant qu'amorces, du nombre de copies de fragments nucléotidiques délimités en position 5' par l'oligonucléotide dégénéré en orientation 5', et en position 3' par l'oligonucléotide dégénéré en orientation 3', et une étape d'identification de la souche de PTLV contenue dans l'échantillon biologique à partir des fragments nucléotidiques amplifiés susmentionnés.

Un objet plus particulièrement décrit consiste en l'utilisation susmentionnée de couples d'oligonucléotides dégénérés tels que définis ci-dessus, caractérisée en ce que lesdits oligonucléotides sont choisis parmi ceux comprenant environ 15 à environ 30 nucléotides issus des séquences nucléotidiques codant pour des fragments protéiques délimités du côté N-terminal par un acide aminé situé entre les positions 75 à 90, et du côté C-terminal par un acide aminé situé entre les positions 230 à 245 des protéines d'enveloppe des différentes souches des PTLV, telles que la protéine d'enveloppe de la souche MT-2 de HTLV-1 représentée par SEQ ID NO : 43, ou la souche NRA de HTLV-2 représenté par SEQ ID NO : 45, ou la souche de STLV-3 représentée par SEQ ID NO : 47, lesdits oligonucléotides dégénérés comprenant un mélange d'oligonucléotides issus de séquences codant pour une région déterminée d'environ 5 à 10 acides aminés des protéines d'enveloppe des différents souches de PTLV, et qui différent entre eux par substitution d'au moins un nucléotide par un autre de manière à ce que chaque oligonucléotide soit susceptible de coder pour la région déterminée susmentionnée issue des fragments protéiques des protéines d'enveloppe des différentes souches des PTLV, telles que la protéine d'enveloppe de la souche MT-2 de HTLV-1, ou la souche NRA de HTLV-2, ou la souche de STLV-3 susmentionnées.

Un objet également plus particulièrement décrit consiste en l'utilisation susmentionnée de couples d'oligonucléotides dégénérés tels que définis ci-dessus, comprenant environ 15 à environ 30 nucléotides issus de séquences nucléotidiques codant pour des fragments polypeptidiques d'environ 5 à environ 10 acides aminés issus de fragments protéiques délimités par les acides aminés situés aux positions 80 à 245, et plus particulièrement aux positions 83 à 241, de la protéine d'enveloppe de la souche MT-2 de HTLV-1 (Gray et al., 1990, Virology, 177: 391-395 ; n° d'accès Genbank M37747) représentée par SEQ ID NO : 43.

Un objet plus particulièrement décrit consiste encore en l'utilisation susmentionnée de couples d'oligonucléotides dégénérés tels que définis ci-dessus, issus de séquences nucléotidiques codant pour les fragments polypeptidiques 83-88, 140-145, 222-228, et 237-241, de la protéine d'enveloppe de la souche MT-2 de HTLV-1, à savoir les fragments suivants :
83-YL/VFPHW-88
140 - NFTQ/REV -145
222-NYS/TCVMVC-228
237-WHVLY-241

Un objet plus particulièrement décrit consiste encore en l'utilisation susmentionnée, d'oligonucléotides dégénérés en orientation 5' issus du brin ADN (+) codant pour :
- le fragment polypeptidique 83-88 de la protéine d'enveloppe de la souche MT-2 de HTLV-1, lesdits oligonucléotides étant choisis parmi ceux de formule (I) suivante :

| | | |
|---|---|---|
| TAYBTNTTYCCNCAYTGG | (I) | SEQ ID NO : 5 |

dans laquelle :
Y représente C ou T,
B représente C, G ou T,
N représente A, C, G ou T,
telles que les amorces oligonucléotidiques 5' choisies parmi les suivantes :

| | | |
|---|---|---|
| PTLVES'83a | TAYBTNTTYCCNCACTGG | SEQ ID NO : 6 |
| PTLVE5'83b | TAYBTNTTYCCNCATTGG | SEQ ID NO : 7 |

Y, B et N étant tels que définis ci-dessus,
- ou le fragment polypeptidique 140-145 de la protéine d'enveloppe de la souche MT-2 de HTLV-1, lesdits oligonucléotides étant choisis parmi ceux de formule (II) suivante :

| | | |
|---|---|---|
| AAYTTYACNCARGARGT | (II) | SEQ ID NO : 8 |

dans laquelle :
Y représente C ou T,
R représente A ou G,
N représente A, C, G ou T,
telles que les amorces oligonucléotidiques 5' choisies parmi les suivantes :

| | | |
|---|---|---|
| PTLVE5'140a | AAYTTYACNCAAGAAGT | SEQ ID NO : 9 |
| PTLVE5'140b | AAYTTYACNCAGGAAGT | SEQ ID NO : 10 |
| PTLVF-5'140c | AAYTTYACNCAAGAGGT | SEQ ID NO : 11 |
| PTLVE5'140d | AAYTTYACNCAGGAGGT | SEQ ID NO : 12 |

Y et N étant tels que définis ci-dessus.

Un objet plus particulièrement décrit consiste encore en l'utilisation susmentionnée, d'oligonucléotides dégénérés en orientation 3' issus du brin ADN (-) codant pour :
- le fragment polypeptidique 140-145 de la protéine d'enveloppe de la souche MT-2 de HTLV-1, lesdits oligonucléotides étant choisis parmi ceux de formule (III) suivante :

| | | |
|---|---|---|
| NACYTCYTGNGTRAARTT | (III) | SEQ ID NO : 13 |

dans laquelle:
Y représente C ou T,
R représente A ou G,
N représente A, C, G ou T,
telles que les amorces oligonucléotidiques 3' choisies parmi les suivantes :

| | | |
|---|---|---|
| PTLVE3'145a | NACYTCYTGNGTAAAATT | SEQ ID NO : 14 |
| PTLVE3'145b | NACYTCYTGNGTGAAATT | SEQ ID NO : 15 |
| PTLVE3'145c | NACYTCYTGNGTAAAGTT | SEQ ID NO : 16 |
| PTLVE3'145d | NACYTCYTGNGTGAAGTT | SEQ ID NO : 17 |

Y et N étant tels que définis ci-dessus,
- ou le fragment polypeptidique 222-228 de la protéine d'enveloppe de la souche MT-2 de HTLV- 1, lesdits oligonucléotides étant choisis parmi ceux de formule (IV) suivante :

| | | |
|---|---|---|
| RMNACNATRCANSWRTARTT | (IV) | SEQ ID NO : 18 |

dans laquelle :
R représente A ou G,
M représente A ou C,
S représente C ou G,
W représente A ou T,
N représente A, C, G ou T,
telles que les amorces oligonucléotidiques 3' choisies parmi les suivantes :

| | | |
|---|---|---|
| PTLVE3'228a | RMNACNATRCANSAATAATT | SEQ ID NO : 19 |
| PTLVE3'228b | RMNACNATRCANSAGTAATT | SEQ ID NO : 20 |
| PTLVE3'228c | RMNACNATRCANSAATAGTT | SEQ ID NO : 21 |
| PTLVE3'228d | RMNACNATRCANSAGTAGTT | SEQ ID NO : 22 |
| PTLVE3'228e | RMNACNATRCANSTATAATT | SEQ ID NO : 23 |
| PTLVE3'228f | RMNACNATRCANSTGTAATT | SEQ ID NO : 24 |
| PTLVE3'228g | RMNACNATRCANSTATAGTT | SEQ ID NO : 25 |
| PTLVE3'228h | RMNACNATRCANSTGTAGTT | SEQ ID NO : 26 |

R, M, S, et N étant tels que définis ci-dessus,
- ou le fragment polypeptidique 237-241 de la protéine d'enveloppe de la souche MT-2 de HTLV-1, lesdits oligonucléotides étant choisis parmi ceux de formule (V) suivante :

| | | |
|---|---|---|
| RTANARNACRTGCCA | (V) | SEQ ID NO : 27 |

dans laquelle :
R représente A ou G,
N représente A, C, G ou T,
telles que les amorces oligonucléotidiques 3' choisies parmi les suivantes :

| | | |
|---|---|---|
| PTLVE3'241a | RTANARNACATGCCA | SEQ ID NO : 28 |
| PTLVE3'241b | RTANARNACGTGCCA | SEQ ID NO : 29 |

R, et N étant tels que définis ci-dessus.

L'utilisation susmentionnées d'oligonucléotides tels que définis ci-dessus, comportant à leur extrémité 5' une séquence comprenant un site de restriction, tels que les sites EcoRI, de séquence GAATTC, ou BamHI, de séquence GGATCC, est également décrite.

A ce titre, est plus particuliérement décrite l'utilisation susmentionnée d'oligonucléotides tel que définis ci-dessus, caractérisés en ce que les oligonucléotides 5' issus du brin ADN (+) correspondant aux fragments polypeptidiques 83-88 ou 140-145 comprennent en 5' une séquence GGAAGAATTC, et en ce que les oligonucléotides 3' issus du brin ADN (-) correspondant aux fragments polypeptidiques 140-145, 222-228, et 237-241 comprennent en 5' une séquence GGAAGGATCC.

Est également décrite l'utilisation susmentionnée d'oligonucléotides tels que définis ci-dessus en tant que sondes, le cas échéant marquées, pour la mise en oeuvre de procédés de détection susmentionnés de PTLV et de souches apparentées.

Est également décrite l'utilisation susmentionnée d'oligonucléotides tels que définis ci-dessus, en tant que couples d'amorces nucléotidiques pour la mise en oeuvre de réactions de polymérisation en chaîne (PCR) pour la détection de toute souche de PTLV, ainsi que de toute souche de virus comportant des séquences apparentées aux SU des PTLV.

Est plus particulièrement décrite l'utilisation susmentionnée de couples d'amorces choisis de telle manière que :
- les oligonucléotides dégénérés 5' correspondent à un mélange d'oligonucléotides 5' issus d'une même région nucléotidique déterminée comprenant environ 15 à environ 30 nucléotides issus du brin ADN (+) et codant pour des fragments polypeptidiques d'environ 5 à environ 10 acides aminés issus de fragments protéiques délimités du côté N-terminal par un acide aminé situé entre les positions 75 à 90, et du côté C-terminal par un acide aminé situé entre les positions 135 à 150 des protéines d'enveloppe des différentes souches des PTLV, notamment codant pour des fragments polypeptidiques d'environ 5 à environ 10 acides aminés issus du fragment protéique délimité du côté N-terminal par l'acide aminé situé à la position 83 et du côté C-terminal par l'acide aminé situé à la position 145 de la protéine d'enveloppe de la souche MT-2 de HTLV-1, lesdits oligonucléotides 5' étant tels qu'ils différent entre eux par substitution d'au moins un.nucléotide par un autre de manière à ce que chaque oligonucléotide soit susceptible de coder pour la région déterminée susmentionnée issue des fragments protéiques des protéines d'enveloppe des différentes souches des PTLV, telles que la protéine d'enveloppe de la souche MT-2 de HTLV-1, ou la souche NRA de HTLV-2, ou la souche de STLV-3 susmentionnées,
- les oligonucléotides dégénérés 3' correspondent à un mélange d'oligonucléotides 3' issus d'une même région nucléotidique déterminée comprenant environ 15 à environ 30 nucléotides issus du brin ADN (-) et codant pour des fragments polypeptidiques d'environ 5 à environ 10 acides aminés issus de fragments protéiques délimités du côté N-terminal par un acide aminé situé entre les positions 125 à 145, et du côté C-terminal par un acide aminé situé entre les positions 230 à 245 des protéines d'enveloppe des différentes souches des PTLV, notamment codant pour des fragments polypeptidiques d'environ 5 à environ 10 acides aminés issus du fragment protéique délimité du côté N-terminal par l'acide aminé situé à la position 140 et du côté C-terminal par l'acide aminé situé à la position 241 de la protéine d'enveloppe de la souche MT-2 de HTLV-1, lesdits oligonucléotides 3' étant tels qu'ils différent entre eux par substitution d'au moins un nucléotide par un autre de manière à ce que chaque oligonucléotide soit susceptible de coder pour la région déterminée susmentionnée issue des fragments protéiques des protéines d'enveloppe des différentes souches des PTLV, telles que la protéine d'enveloppe de la souche MT-2 de HTLV-1, ou la souche NRA de HTLV-2, ou la souche de STLV-3 susmentionnées,
étant bien entendu que lesdites amorces 5' et 3' susmentionnées ne peuvent pas être complémentaires l'une de l'autre.

L'utilisation susmentionnée de couples d'oligonucléotides dégénérés tels que définis ci-dessus, caractérisée en ce que les oligonucléotides dégénérés 5' sont choisis parmi les oligonucléotides 5' de formules (I) et (II) susmentionnées, et en ce que les oligonucléotides dégénérés 3' sont choisis parmi les oligonucléotides 3' de formules (III) à (V) susmentionnées, est plus particulièrement concernée.

Est plus particulièrement concernée l'utilisation susmentionnée de couples d'amorces tels que définis ci-dessus, caractérisée en ce que l'amorce 5' est choisie parmi les oligonucléotides 5' issus du brin ADN (+) correspondant aux fragments polypeptidiques 83 - 88 ou 140-145 définis ci-dessus, telles que les amorces PTLVE 5'83 a et b et PTLVE 5' 140 a à d susmentionnés, et en ce que l'amorce 3' est choisie parmi les oligonucléotides 3' issus du brin ADN (-) correspondant aux fragments polypeptidiques 140 - 145, 222 - 228 ou 237 - 241 définis ci-dessus, telles que les amorces PTLVE 3'145 a à d, PTLV3'228 a à h, et PTLVE 3 '145 a à d, PTLV 3'228 a à h, et PTLVE 3'241 a et b susmentionnées.

Un objet est plus particulièrement l'utilisation susmentionnée de couples d'oligonucléotides dégénérés tels que définis ci-dessus, lesdits oligonucléotides étant choisis de manière à ce qu'ils permettent l'amplification, à partir d'un échantillon biologique susceptible de contenir de l'ADN de PTLV, de séquences nucléotidiques codant pour des fragments protéiques comprenant une séquence délimitée du côté N-terminal par l'acide aminé situé en position 89, et du côté C-terminal par l'acide aminé situé en position 139 de la protéine d'enveloppe de la souche MT-2 de HTLV-1 représentée par SEQ ID NO : 43, ou comprenant une séquence analogue comprise dans la protéine d'enveloppe d'une autre souche de PTLV que HTLV-1, telle que la séquence délimitée du côté N-terminal par l'acide aminé situé en position 85, et du côté C-terminal par l'acide aminé situé en position 135 de la protéine d'enveloppe de la souche NRA de HTLV-2 représentée par SEQ ID NO : 45, ou la séquence délimitée du côté N-terminal par l'acide aminé situé en position 88, et du côté C-terminal par l'acide aminé situé en position 144 de la protéine d'enveloppe de la souche de STLV-3 représentée par SEQ ID NO : 47.

Un objet est plus particulièrement encore l'utilisation susmentionnée de couples d'oligonucléotides dégénérés tels que définis ci-dessus, caractérisée en ce que les oligonucléotides dégénérés 5' sont choisis parmi les oligonucléotides 5' de formule (I) susmentionnée, et en ce que les oligonucléotides dégénérés 3' sont choisis parmi les oligonucléotides 3' de formules (III) à (V) susmentionnées.

Est concernée plus particulièrement l'utilisation susmentionnée de couples d'oligonucléotides dégénérés tels que définis ci-dessus, caractérisée en ce que :
- les oligonucléotides dégénérés 5' sont ceux de formule (I) suivante :

| | | |
|---|---|---|
| PTLVE5'83b | TAYBTNTTYCCNCATTGG | SEQ ID NO : 5 |

Y, B et N étant tels que définis ci-dessus,
- les oligonucléotides dégénérés 3' sont ceux de formule (III) suivante :

| | | |
|---|---|---|
| PTLVE3'145a | NACYTCYTGNGTAAAATT | SEQ ID NO : 14 |

Y et N étant tels que définis ci-dessus.

Les oligonucléotides tels que définis ci-dessus, en tant que tels, sont également concernés.

A ce titre, sont décrits plus particulièrement les oligonucléotides tels que définis ci-dessus, correspondants :
- aux oligonucléotides dégénérés en orientation 5' issus du brin ADN (+) codant pour :
* le fragment polypeptidique 83-88 de la protéine d'enveloppe de la souche MT-2 de HTLV-1, lesdits oligonucléotides étant choisis parmi ceux de formule (I) suivante :

| | | |
|---|---|---|
| TAYBTNTTYCCNCAYTGG | (I) | SEQ ID NO : 5 |

dans laquelle :
Y représente C ou T,
B représente C, G ou T,
N représente A, C, G ou T,
telles que les amorces oligonucléotidiques 5' choisies parmi les suivantes :

| | | |
|---|---|---|
| PTLVE5'83a | TAYBTNTTYCCNCACTGG | SEQ ID NO : 6 |
| PTLVE5'83b | TAYBTNTTYCCNCATTGG | SEQ ID NO : 7 |

Y, B et N étant tels que définis ci-dessus,
* le fragment polypeptidique 140-145 de la protéine d'enveloppe de la souche MT-2 de HTLV-1, lesdits oligonucléotides étant choisis parmi ceux de formule (II) suivante:

| | | |
|---|---|---|
| AAYTTYACNCARGARGT | (II) | SEQ ID NO : 8 |

dans laquelle :
Y représente C ou T,
R représente A ou G,
N représente A, C, G ou T,
telles que les amorces oligonucléotidiques 5' choisies parmi les suivantes :

| | | |
|---|---|---|
| PTLVE5'140a | AAYTTYACNCAAGAAGT | SEQ ID NO : 9 |
| PTLVE5'140b | AAYTTYACNCAGGAAGT | SEQ ID NO : 10 |
| PTLVE5'140c | AAYTTYACNCAAGAGGT | SEQ ID NO : 1 |
| PTLVE5'140d | AAYTTYACNCAGGAGGT | SEQ ID NO : 12 |

Y et N étant tels que définis ci-dessus,
- aux oligonucléotides dégénérés en orientation 3' issus du brin ADN (-) codant pour :
* le fragment polypeptidique 140-145 de la protéine d'enveloppe de la souche MT-2 de HTLV-1, lesdits oligonucléotides étant choisis parmi ceux de formule (III) suivante :

| | | |
|---|---|---|
| NACYTCYTGNGTRAARTT | (III) | SEQ ID NO : 13 |

dans laquelle :
Y représente C ou T,
R représente A ou G,
N représente A, C, G ou T,
telles que les amorces oligonucléotidiques 3' choisies parmi les suivantes :

| | | |
|---|---|---|
| PTLVE3'145a | NACYTCYTGNGTAAAATT | SEQ ID NO : 14 |
| PTLVE3'145b | NACYTCYTGNGTGAAATT | SEQ ID NO : 15 |
| PTLVE3'145c | NACYTCYTGNGTAAAGTT | SEQ ID NO : 16 |
| PTLVE3'145d | NACYTCYTGNGTGAAGTT | SEQ ID NO : 17 |

Y et N étant tels que définis ci-dessus,
* le fragment polypeptidique 222-228 de la protéine d'enveloppe de la souche MT-2 de HTLV-1, lesdits oligonucléotides étant choisis parmi ceux de formule (IV) suivante :

| | | |
|---|---|---|
| RMNACNATRCANSWRTARTT | (IV) | SEQ ID NO : 18 |

dans laquelle :
R représente A ou G,
M représente A ou C,
S représente C ou G,
W représente A ou T,
N représente A, C, G ou T,
telles que les amorces oligonucléotidiques 3' choisies parmi les suivantes :

| | | |
|---|---|---|
| PTLVE3'228a | RMNACNATRCANSAATAATT | SEQ ID NO : 19 |
| TLVE3'228b | RMNACNATRCANSAGTAATT | SEQ ID NO : 20 |
| PTLVE3'228c | RMNACNATRCANSAATAGTT | SEQ ID NO : 21 |
| PTLVE3'228d | RMNACNATRCANSAGTAGTT | SEQ ID NO : 22 |
| PTLVE3'228e | RMNACNATRCANSTATAATT | SEQ ID NO : 23 |
| PTLVE3'228f | RMNACNATRCANSTGTAATT | SEQ ID NO : 24 |
| PTLVE3'228g | RMNACNATRCANSTATAGTT | SEQ ID NO : 25 |

| | | |
|---|---|---|
| PTLVE3'228h | RMNACNATRCANSTGTAGTT | SEQ ID NO : 26 |

R, M, S, et N étant tels que définis ci-dessus,
* le fragment polypeptidique 237-241 de la protéine d'enveloppe de la souche MT-2 de HTLV-1, lesdits oligonucléotides étant choisis parmi ceux de formule (V) suivante :

| | | |
|---|---|---|
| RTANARNACRTGCCA | (V) | SEQ ID NO : 27 |

dans laquelle :
R représente A ou G,
N représente A, C, G ou T,
telles que les amorces oligonucléotidiques 3' choisies parmi les suivantes :

| | | |
|---|---|---|
| PTLVE3'241a | RTANARNACATGCCA | SEQ ID NO : 28 |
| PTLVE3'241b | RTANARNACGTGCCA | SEQ ID NO : 29 |

R, et N étant tels que définis ci-dessus.

Est également décrit un procédé de détection de toute souche de PTLV, à savoir de toute souche appartenant aux HTLV-1, HTLV-2, STLV-1, STLV-2, et STLV-3, ainsi que de toute souche de virus comportant des séquences apparentées aux SU des PTLV, telles que définies ci-dessus, caractérisé en ce qu'il comprend :
- la mise en contact d'un couple d'oligonucléotides dégénérés 5' et 3' tels que définis ci-dessus, avec l'ADN génomique ou l'ADN complémentaire dérivé à partir d'ARN extraits du contenu d'un échantillon biologique (tels que cellules sanguines, de moelle osseuse, biopsies, notamment de peau ou autres organes, ou frottis) susceptible de contenir des PTLV tels que définis ci-dessus,
- l'amplification de fragments d'ADN codant pour un fragments des protéines d'enveloppe des différentes souches des PTLV tel que défini ci-dessus,
- la détection des fragments d'ADN amplifiés lors de l'étape précédente, cette détection pouvant être corrélée à la détection et le cas échéant à l'identification de PTLV tels que définis ci-dessus dans ledit échantillon biologique.

Est concerné également un procédé de détection de toute souche de PTLV tel que défini ci-dessus, caractérisé en ce que l'étape d'amplification comprend la mise en oeuvre de deux réactions d'amplification, la deuxième réaction étant effectuée sur un échantillon de produits obtenus dans le cadre de la première réaction à l'aide des mêmes oligonucléotides 5' que dans le cas de la première réaction, et d'oligonucléotides 3' différents de ceux utilisés dans la première réaction, à savoir des amorces 3' dites « nichées » hybridant avec une région située plus en amont de la séquence codant pour la SU que les amorces 3' utilisées dans la première réaction.

Est également décrit un procédé de détection de toute souche de PTLV tel que défini ci-dessus, caractérisé en ce qu'il comprend :
- une première réaction d'amplification génique effectuée à l'aide de couples d'oligonucléotides dégénérés choisis parmi les couples :
   * oligonucléotides de formule (I)/oligonucléotides de formule (IV), ou
   * oligonucléotides de formule (I)/oligonucléotides de formule (V), ou
   * oligonucléotides de formule (II)/oligonucléotides de formule (V),
- et une deuxième étape d'amplification du nombre de copies de fragments d'ADN obtenus lors de l'étape précédente à l'aide de couples d'oligonucléotides dégénérés choisis respectivement parmi les couples :
   * oligonucléotides de formule (I)/oligonucléotides de formule (III), ou
   * oligonucléotides de formule (I)/oligonucléotides de formule (ID ou IV), ou
   * oligonucléotides de formule (II)/oligonucléotides de formule (IV),
- la détection des fragments d'ADN amplifiés lors de l'étape précédente, cette détection pouvant être corrélée à la détection et le cas échéant à l'identification de PTLV dans l'échantillon biologique.

Est concerné également un procédé de détection de toute souche de PTLV tel que défini ci-dessus, caractérisé en ce qu'il comprend :
une première réaction d'amplification génique effectuée à l'aide de couples d'oligonucléotides dégénérés choisis de telle manière que :
* les oligonucléotides dégénérés 5' sont ceux de formule (I) suivante :

| | | |
|---|---|---|
| PTLVE5'83b | TAYBTNTTYCCNCATTGG | SEQ ID NO : 5 |

Y, B et N étant tels que définis ci-dessus,
* les oligonucléotides dégénérés 3' sont ceux de formule (V) suivante :

| | | |
|---|---|---|
| PTLVE3'241b | RTANARNACRTGCCA | SEQ ID NO : 29 |

R, et N étant tels que définis ci-dessus,
- une deuxième réaction d'amplification génique effectuée à l'aide de couples d'oligonucléotides dégénérés choisis de telle manière que :
* les oligonucléotides dégénérés 5' sont ceux de formule (I) suivante :

| | | |
|---|---|---|
| PTLVE5'83b | TAYBTNTTYCCNCATTGG | SEQ ID NO : 5 |

Y, B et N étant tels que définis ci-dessus,
* les oligonucléotides dégénérés 3' sont ceux de formule (III) suivante :

| | | |
|---|---|---|
| PTLVE3'145a | NACYTCYTGNGTAAAATT | SEQ ID NO : 14 |

Y et N étant tels que définis ci-dessus.

Est concerné plus particulièrement un procédé de détection tel que défini ci-dessus, caractérisé en ce que l'étape d'amplification est effectuée dans les conditions suivantes :
- dénaturation à 94°C pendant 5 min,
- une première réaction PCR en conditions dites de « touch down » effectuée dans un milieu contenant de la Taq polymérase ou autres ADN polymérases fonctionnant à haute température, cette première réaction PCR comprenant :
   - 15 cycles d'affilée « touch down » variant par la température d'élongation qui diminue de 1°C à chaque cycle comprenant :
      * une étape de dénaturation à 94°C pendant 15 sec,
      * une étape combinant appariement et élongation à une température variant entre 65°C et 50 °C pendant 20 sec,
   - 30 cycles classiques comprenant :
      * une étape de dénaturation à 94°C pendant 15 sec,
      * une étape d'appariement à 50°C pendant 30 sec,
      * une étape d'élongation à 72°C pendant 30 sec,
- une deuxième réaction PCR effectuée sur un échantillon de produits obtenus dans le cadre de la première réaction PCR susmentionnée à l'aide de la même amorce 5' que dans le cas de la réaction PCR précédente, et d'une amorce 3' différente de celle utilisée dans la réaction PCR précédente, à savoir une amorce 3' dite « nichée » hybridant avec une région située plus en amont de la séquence codant pour la SU que l'amorce 3' utilisée à l'étape précédente.

Est plus particulièrement décrit un procédé de détection tel que défini ci-dessus, caractérisé en ce que l'étape de détection, et le cas échéant d'identification, est effectuée dans les conditions suivantes :
- ligation directe des fragments amplifiés lors de l'étape d'amplification dans un plasmide tel que pCR4-TOPO (Invitrogen),
- transformation de bactéries avec le plasmide susmentionné comprenant un gène marqueur tel qu'un gène de résistance à un antibiotique, notamment à la kanamycine,
- repiquage de colonies bactériennes (notamment entre 10 et 100), culture, extraction de l'ADN, et séquençage (notamment à l'aide des amorces universelles T3 ou T7 dans le cas de l'utilisation du vecteur pCR4-TOPO).

Une trousse ou kit pour la mise en oeuvre d'un procédé de détection tel que défini ci-dessus, caractérisé en ce qu'il comprend un couple d'oligonucléotides dégénérés susmentionnés, et, le cas échéant, les réactifs nécessaires à la mise en oeuvre de la réaction d'amplification par PCR et pour la détection des fragments amplifiés, sont également concernés.

L'application du procédé de détection défini ci-dessus au diagnostic de pathologies liées à une infection par un PTLV, ou par un virus comportant des séquences apparentées aux SU des PTLV, chez l'homme ou l'animal, telles que les hémopathies, les maladies auto-immunes, les maladies inflammatoires, les maladies dégénératives, est également décrite.

A ce titre, est décrite toute méthode de diagnostic in vitro de pathologies susmentionnées par mise en oeuvre d'un procédé de détection défini ci-dessus, la détection de fragments d'ADN amplifiés pouvant être corrélée au diagnostic desdites pathologies.

Le cas échéant, les méthodes de diagnostic in vitro décrites comprenant une étape supplémentaire d'identification de PTLV ou virus apparentés aux PTLV présents dans l'échantillon biologique, par séquençage des fragments d'ADN amplifiés.

L'application du procédé de détection défini ci-dessus, au criblage et à l'identification de nouveaux agents infectieux chez l'homme ou l'animal, et plus particulièrement de nouvelles souches (ou variants) de virus susceptibles d'être classés dans les PTLV, ou de virus comportant des séquences apparentées aux SU des PTLV, est également décrite.

Les méthodes de criblage et d'identification susmentionnées de nouveaux agents infectieux sont effectuées par mise en oeuvre d'un procédé de détection défini ci-dessus et comprennent une étape supplémentaire d'identification des nouveaux variants de PTLV ou de virus apparentés par séquençage des fragments d'ADN amplifiés.

Est décrite également l'application du procédé de détection défini ci dessus au criblage de gènes de prédisposition ou de résistance à des pathologies chez l'homme ou l'animal liées à la présence de séquences des PTLV ou de séquences apparentées, ou à une infection par un PTLV, telles que les hémopathies, les maladies auto-immunes, les maladies dégénératives.

L'application du procédé de détection défini ci-dessus, au criblage ou à la conception de nouveaux agents thérapeutiques comprenant des séquences entières ou partielles des protéines d'enveloppe de nouveaux variants de PTLV ainsi détectés, est également décrite.

L'application du procédé de détection tel que défini ci-dessus, au criblage ou à la conception de nouveaux vecteurs de thérapie cellulaire utilisant les propriétés de tropisme des séquences entières ou partielles des protéines d'enveloppe de nouveaux variants de PTLV ainsi détectés, est également décrite.

Les variants de type HTLV-1 tels qu'obtenus par mise en oeuvre d'un procédé de détection défini ci-dessus sont décrits, correspondants :
- au variant dont la protéine d'enveloppe est telle qu'elle comprend la séquence peptidique SEQ ID NO : 31 suivante : à savoir une séquence correspondant à la séquence délimitée par les acides aminés situés aux positions 89 à 139 de la protéine d'enveloppe de la souche MT-2 de HTLV-1, dans laquelle le résidu arginine (R) en position 94, et le résidu sérine (S) en position 101, sont respectivement remplacés par un résidu proline (P) et un résidu leucine (L) indiqués en gras et soulignés,
   et dont la séquence nucléotidique codant pour sa protéine d'enveloppe est telle qu'elle comprend la séquence SEQ ID NO : 30 suivante : à savoir une séquence correspondant à la séquence délimitée par les nucléotides situés aux positions 265 à 417 de la séquence codant pour la protéine d'enveloppe de la souche MT-2 de HTLV-1, dans laquelle G en position 281, C en position 302, et G en position 333, sont respectivement remplacés par C, T, et A indiqués en gras et soulignés.
- au variant dont la protéine d'enveloppe est telle qu'elle comprend la séquence peptidique SEQ ID NO : 33 suivante : à savoir une séquence correspondant à la séquence délimitée par les acides aminés situés aux positions 89 à 139 de la protéine d'enveloppe de la souche MT-2 de HTLV-1, dans laquelle le résidu isoleucine (I) en position 89, et le résidu sérine (S) en position 101, sont respectivement remplacés par un résidu valine (V) et un résidu leucine (L) indiqués en gras et soulignés,
   et dont la séquence nucléotidique codant pour sa protéine d'enveloppe est telle qu'elle comprend la séquence SEQ ID NO : 32 suivante : à savoir une séquence correspondant à la séquence délimitée par les nucléotides situés aux positions 265 à 417 de la séquence codant pour la protéine d'enveloppe de la souche MT-2 de HTLV-1, dans laquelle A en position 266, C en position 302, et G en position 333, sont respectivement remplacés par G, T, et A indiqués en gras et soulignés.
- au variant dont la protéine d'enveloppe est telle qu'elle comprend la séquence peptidique SEQ ID NO : 35 suivante : à savoir une séquence correspondant à la séquence délimitée par les acides aminés situés aux positions 89 à 139 de la protéine d'enveloppe de la souche MT-2 de HTLV-1, dans laquelle le résidu sérine (S) en position 101, est remplacé par un résidu leucine (L) indiqué en gras et souligné,
   et dont la séquence nucléotidique codant pour sa protéine d'enveloppe est telle qu'elle comprend la séquence SEQ ID NO : 34 suivante : à savoir une séquence correspondant à la séquence délimitée par les nucléotides situés aux positions 265 à 417 de la séquence codant pour la protéine d'enveloppe de la souche MT-2 de HTLV-1, dans laquelle C en position 302, G en position 333, et G en position 408, sont respectivement remplacés par T, A, et A indiqués en gras et soulignés.
- au variant dont la protéine d'enveloppe est telle qu'elle comprend la séquence peptidique SEQ ID NO : 37 suivante : à savoir une séquence correspondant à la séquence délimitée par les acides aminés situés aux positions 89 à 139 de la protéine d'enveloppe de la souche MT-2 de HTLV-1, dans laquelle le résidu alanine (A) en position 127, est remplacé par un résidu proline (P) indiqué en gras et souligné,
   et dont la séquence nucléotidique codant pour sa protéine d'enveloppe est telle qu'elle comprend la séquence SEQ ID NO : 36 suivante : à savoir une séquence correspondant à la séquence délimitée par les nucléotides situés aux positions 265 à 417 de la séquence codant pour la protéine d'enveloppe de la souche MT-2 de HTLV-1, dans laquelle G en position 379, est remplacé par C indiqué en gras et souligné,
- au variant dont la protéine d'enveloppe est telle qu'elle comprend la séquence peptidique SEQ ID NO : 39 suivante : à savoir une séquence correspondant à la séquence délimitée par les acides aminés situés aux positions 89 à 145 de la protéine d'enveloppe de la souche MT-2 de HTLV-1, dans laquelle le résidu tyrosine (Y) en position 100, et le résidu thréonine (T) en position 125, sont respectivement remplacés par un résidu histidine (H) et un résidu alanine (A) indiqués en gras et soulignés,
   et dont la séquence nucléotidique codant pour sa protéine d'enveloppe est telle qu'elle comprend la séquence SEQ ID NO : 38 suivante : à savoir une séquence correspondant à la séquence délimitée par les nucléotides situés aux positions 265 à 435 de la séquence codant pour la protéine d'enveloppe de la souche MT-2 de HTLV-1, dans laquelle T en position 298, A en position 373, T en position 426, A en position 429, et T en position 435, sont respectivement remplacés par C, G, C, G, et A indiqués en gras et soulignés.

Le variant de type HTLV-2 tel qu'obtenu par mise en oeuvre d'un procédé de détection défini ci-dessus est également décrit, caractérisé en ce que:
- sa protéine d'enveloppe est telle qu'elle comprend la séquence peptidique SEQ ID NO : 41 suivante : à savoir une séquence correspondant à la séquence délimitée par les acides aminés situés aux positions 85 à 135 de la protéine d'enveloppe de la souche prototype NRA de HTLV-2 (décrite par Lee et al., 1993. Virology 196, 57-69 ; n° d'accès Genbank L20734.1), dans laquelle les résidus lysine (K) en position 86, cystéine (C) en position 113, glycine (G) en position 122, sérine (S) en position 126, et lysine (K) en position 130, sont respectivement remplacés par les résidus arginine (R), sérine (S), alanine (A), thréonine (T), et asparagine (N) indiqués en gras et soulignés,
- la séquence nucléotidique codant pour sa protéine d'enveloppe est telle qu'elle comprend la séquence SEQ ID NO : 40 suivante : à savoir une séquence correspondant à la séquence délimitée par les nucléotides situés aux positions 253 à 405 de la séquence codant pour la protéine d'enveloppe de la souche NRA de HTLV-2, dans laquelle A en position 257, G en position 258, T en position 267, A en position 282, C en position 294, T en position 300, A en position 333, G en position 338, G en position 365, G en position 377, G en position 390, et C en position 396, sont respectivement remplacés par G, A, C, G, T, C, G, C, C, C, T, et T indiqués en gras et soulignés.

Sont concernés également les polypeptides délimités du côté N-terminal par un acide aminé situé entre les positions 75 à 90, et du côté C-terminal par un acide aminé situé entre les positions 230 à 245 des protéines d'enveloppe des différentes souches des PTLV, telles que la protéine d'enveloppe de la souche MT-2 de HTLV-1 représentée par SEQ ID NO : 43, ou de la souche NRA de HTLV-2 représentée par SEQ ID NO : 45, ou de la souche de STLV-3 représentée par SEQ ID NO : 47, ou de virus portant des séquences apparentées aux SU des PTLV, ou délimités du côté N-terminal par un acide aminé situé entre les positions 75 à 90, et du côté C-terminal par un acide aminé situé entre les positions 135 à 150 desdites protéines d'enveloppe des différentes souches des PTLV.

Sont également décrits les polypeptides définis ci-dessus, choisis parmi :
- le polypeptide délimité du côté N-terminal par l'acide aminé situé à la position 83 ou 89, et du côté C-terminal par l'acide aminé situé à la position 139 ou 145, de la protéine d'enveloppe de la souche MT-2 de HTLV-1 représentée par SEQ ID NO : 43,
- le polypeptide délimité du côté N-terminal par l'acide aminé situé à la position 79 ou 85, et du côté C-terminal par l'acide aminé situé à la position 135 ou 141, de la protéine d'enveloppe de la souche NRA de HTLV-2 représentée par SEQ ID NO : 45,
- le polypeptide délimité du côté N-terminal par l'acide aminé situé à la position 82 ou 88, et du côté C-terminal par l'acide aminé situé à la position 138 ou 144, de la protéine d'enveloppe de la souche de STLV-3 représentée par SEQ ID NO : 47.

Sont concernés également les polypeptides codés par les fragments d'ADN amplifiés dans le cadre du procédé de détection défini ci-dessus, des variants de type HTLV-1 à HTLV-2 susmentionnés, caractérisés en ce qu'ils comprennent les séquences peptidiques suivantes :
- polypeptide 1 (SEQ ID NO : 31) : à savoir une séquence correspondant à la séquence délimitée par les acides aminés situés aux positions 89 à 139 de la protéine d'enveloppe de la souche MT-2 de HTLV-1, dans laquelle le résidu arginine (R) en position 94, et le résidu sérine (S) en position 101, sont respectivement remplacés par un résidu proline (P) et un résidu leucine (L) indiqués en gras et soulignés,
- polypeptide 2 (SEQ ID NO : 33): à savoir une séquence correspondant à la séquence délimitée par les acides aminés situés aux positions 89 à 139 de la protéine d'enveloppe de la souche MT-2 de HTLV-1, dans laquelle le résidu isoleucine (I) en position 89, et le résidu sérine (S) en position 101, sont respectivement remplacés par un résidu valine (V) et un résidu leucine (L) indiqués en gras et soulignés,
- polypeptide 3 (SEQ ID NO : 35): à savoir une séquence correspondant à la séquence délimitée par les acides aminés situés aux positions 89 à 139 de la protéine d'enveloppe de la souche MT-2 de HTLV-1, dans laquelle le résidu sérine (S) en position 101, est remplacé par un résidu leucine (L) indiqué en gras et souligné.
- polypeptide 4 (SEQ ID NO : 37): à savoir une séquence correspondant à la séquence délimitée par les acides aminés situés aux positions 89 à 139 de la protéine d'enveloppe de la souche MT-2 de HTLV-1, dans laquelle le résidu alanine (A) en position 127, est remplacé par un résidu proline (P) indiqué en gras et souligné,
- polypeptide 5 (SEQ ID NO : 39) : à savoir une séquence correspondant à la séquence délimitée par les acides aminés situés aux positions 89 à 145 de la protéine d'enveloppe de la souche MT-2 de HTLV-1, dans laquelle le résidu tyrosine (Y) en position 100, et le résidu thréonine (T) en position 125, sont respectivement remplacés par un résidu histidine (H) et un résidu alanine (A) indiqués en gras et soulignés,
- polypeptide 6 (SEQ ID NO : 41) : à savoir une séquence correspondant à la séquence délimitée par les acides aminés situés aux positions 85 à 135 de la protéine d'enveloppe de la souche prototype NRA de HTLV-2, dans laquelle les résidus lysine (K) en position 86, cystéine (C) en position 113, glycine (G) en position 122, sérine (S) en position 126, et lysine (K) en position 130, sont respectivement remplacés par les résidus arginine (R), sérine (S), alanine (A), thréonine (T), et asparagine (N) indiqués en gras et soulignés.

Sont également décrits les acides nucléiques caractérisés en ce qu'ils codent pour un polypeptide tel que défini ci-dessus.

Sont concernés plus précisément les acides nucléiques susmentionnés, comprenant les séquences nucléotidiques suivantes :
- acide nucléique 1 a (SEQ ID NO : 30): à savoir une séquence correspondant à la séquence délimitée par les nucléotides situés aux positions 265 à 417 de la séquence codant pour la protéine d'enveloppe de la souche MT-2 de HTLV-1, dans laquelle G en position 281, C en position 302, et G en position 333, sont respectivement remplacés par C, T, et A indiqués en gras et soulignés,
ou toute séquence nucléotidique dérivée par dégénérescence du code génétique et codant pour le polypeptide 1 susmentionné,
- acide nucléique 2 a (SEQ ID NO : 32): à savoir une séquence correspondant à la séquence délimitée par les nucléotides situés aux positions 265 à 417 de la séquence codant pour la protéine d'enveloppe de la souche MT-2 de HTLV-1, dans laquelle A en position 266, C en position 302, et G en position 333, sont respectivement remplacés par G, T, et A indiqués en gras et soulignés,
ou toute séquence nucléotidique dérivée par dégénérescence du code génétique et codant pour le polypeptide 2 susmentionné,
- acide nucléique 3 a (SEQ ID NO : 34) : à savoir une séquence correspondant à la séquence délimitée par les nucléotides situés aux positions 265 à 417 de la séquence codant pour la protéine d'enveloppe de la souche MT-2 de HTLV-1, dans laquelle C en position 302, G en position 333, et G en position 408, sont respectivement remplacés par T, A, et A indiqués en gras et soulignés,
ou toute séquence nucléotidique dérivée par dégénérescence du code génétique et codant pour le polypeptide 3 la revendication 24,
- acide nucléique 4 a (SEQ ID NO : 36): à savoir une séquence correspondant à la séquence délimitée par les nucléotides situés aux positions 265 à 417 de la séquence codant pour la protéine d'enveloppe de la souche MT-2 de HTLV-1, dans laquelle G en position 379, est remplacé par C indiqué en gras et souligné,
   ou toute séquence nucléotidique dérivée par dégénérescence du code génétique et codant pour le polypeptide 4 susmentionné,
- acide nucléique 5 a (SEQ ID NO : 38) : à savoir une séquence correspondant à la séquence délimitée par les nucléotides situés aux positions 265 à 435 de la séquence codant pour la protéine d'enveloppe de la souche MT-2 de HTLV-1, dans laquelle T en position 298, A en position 373, T en position 426, A en position 429, et T en position 435, sont respectivement remplacés par C, G, C, G, et A indiqués en gras et soulignés.
ou toute séquence nucléotidique dérivée par dégénérescence du code génétique et codant pour le polypeptide 5 susmentionné,
- acide nucléique 6 a (SEQ ID NO : 40): à savoir une séquence correspondant à la séquence délimitée par les nucléotides situés aux positions 253 à 405 de la séquence codant pour la protéine d'enveloppe de la souche NRA de HTLV-2, dans laquelle A en position 257, G en position 258, T en position 267, A en position 282, C en position 294, T en position 300, A en position 333, G en position 338, G en position 365, G en position 377, G en position 390, et C en position 396, sont respectivement remplacés par G, A, C, G, T, C, G, C, C, C, T, et T indiqués en gras et soulignés,
ou toute séquence nucléotidique dérivée par dégénérescence du code génétique et codant pour le polypeptide 6 susmentionné.

Sont concernés également les anticorps polyclonaux ou monoclonaux dirigés contre un nouveau variant de type HTLV - 1 ou HTLV - 2 tel que défini ci-dessus, ou contre un polypeptide défini ci-dessus, lesdits anticorps étant tels qu'obtenus par immunisation d'un animal approprié avec un polypeptide susmentionné.

Est également décrite toute composition pharmaceutique, notamment vaccins ou vecteurs thérapeutiques, conçus à partir des variants de type HTLV-1 ou HTLV-2 tels que définis ci-dessus, et plus particulièrement toute composition pharmaceutique comprenant un polypeptide tel que défini ci-dessus, notamment les polypeptides 1 à 6 définis ci-dessus, ou un acide nucléique la à 6a défini ci-dessus, ou des anticorps susmentionnés, le cas échéant en association avec un véhicule pharmaceutiquement acceptable.

Est concernée également l'utilisation des variants de type HTLV-1 ou HTLV-2 tels que définis ci-dessus, ou des polypeptides tels que définis ci-dessus, notamment des polypeptides 1 à 6, ou des acides nucléiques la à 6a définis ci-dessus, ou des anticorps susmentionnés, pour la préparation de médicaments destinés à la prévention ou au traitement des infections d'un individu par les PTLV susmentionnés, ainsi que des pathologies définies ci-dessus liées à l'infection par ces PTLV.

L'invention sera davantage illustrée à l'aide de la description détaillée qui suit de l'obtention d'amorces selon l'invention et de leur utilisation pour la détection de nouveaux variants de HTLV.

### I. MISE AU POINT D'OUTILS MOLECULAIRES ET DE STRATEGIES DE DETECTION DE SEQUENCES PAN-PTLV PAR AMPLIFICATION, CLONAGE ET SEQUENÇAGE, DE SEQUENCES NUCLEOTIDIQUES APPARENTEES AUX SU DES ENVELOPPES PTLV.

### 1. Recherche de motifs peptidiques conservés en N-terminus des SU de PTLV

La question principale résolue par les inventeurs est la mise au point d'outils et d'une méthode permettant d'amplifier, de cloner et d'identifier toute séquence nucléotidique apparentée à la SU des PTLV qui est responsable de la reconnaissance de leur récepteur cellulaire (Kim et coll, 2000). Pour cela, les inventeurs ont cherché des motifs peptidiques conservés dans la SU des enveloppes de PTLV pour en déduire des séquences nucléotidiques permettant de les représenter toutes. Ces motifs peptidiques devaient répondre idéalement aux 5 critères suivants, selon leur importance décroissante:
- Etre conservés parmi la plupart, sinon la totalité, des séquences déjà décrites d'enveloppes de PTLV. Une telle conservation serait une garantie de leur efficacité potentielle dans la détection de nouvelles séquences de type PTLV.
- Représenter au moins 5 acides aminés conservés de la SU des PTLV, afin d'en dériver une séquence minimale de 15 nucléotides. En effet, étant donnée la complexité des génomes eucaryotes, ce minimum de 15 nucléotides est requis pour permettre la détection spécifique d'une séquence nucléotidique donnée.
- Permettre l'amplification de séquences situées en amont du motif C I/M V C qui est conservé dans la SU des PTLV et semble analogue au motif CWLC décrit dans la SU des MuLV (Sitbon et coll, 1991). Ce motif semble en effet, marquer une région charnière entre, en son amont, la partie de la SU responsable de la reconnaissance du récepteur et, en son aval, les domaines carboxy terminaux de la SU impliqués dans l'association avec la TM et des étapes de l'entrée virale postérieures à la reconnaissance du récepteur (Battini, et coll, 1992; Battini et coll, 1995; Lavillette et coll, 1998; Kim et coll, 2000; Lavillette et coll, 2001). Etre suffisamment distants les uns des autres pour permettre l'amplification d'un fragment dont la longueur augmenterait les chances de détection d'un polymorphisme éventuel entre différentes séquences.
- Etre situés de façon à permettre deux réactions d'amplifications d'ADN successives, dont la deuxième, nichée, est réalisée à partir des produits de la première amplification, et permettrait l'amplification d'un fragment interne au premier fragment amplifié. Cette amplification nichée permettrait d'augmenter la probabilité d'amplification d'un fragment qui corresponde bien à une séquence apparentée à la SU des PTLV.

Selon ces critères, les inventeurs ont identifié les motifs d'acides aminés suivants, présents dans toutes ou la quasi-totalité des SU connues de PTLV, et pouvant se prêter au développement de cette stratégie :
- Motif peptidique 1 : Y L / V F P H W
- Motif peptidique 2: N F T Q/R E V
- Motif peptidique 3 : N Y S /T C I /M V C
- Motif peptidique 4 : W H V L Y

### 2. Oligonucléotides dégénérés de synthèse correspondant aux motifs conservés dans la partie amino terminale de la SU des PTLV

À partir des séquences d'acides aminés des motifs peptidiques conservés identifiés ci-dessus et suivant la correspondance nucléotidique en application du code génétique eucaryote, les inventeurs ont déterminé des séquences nucléotidiques dégénérées (SND) qui ont servi de base pour la conception d'oligonucléotides de synthèse (OS). Plusieurs critères ont présidé à la conception d'OS correspondants à ces SND :
- Lorsque la multiplication des positions dégénérées dans une SND faisait que la complexité de l'OS correspondant dépasse 512 oligonucléotides dans-le mélange de synthèse, la synthèse d'OS supplémentaires pour cette SND est alors effectuée pour lever une partie de cette complexité.
- La synthèse d'un ou 2 OS supplémentaires, dans la limite de 4 OS par SND, est effectuée même pour des complexités inférieures à 512, lorsque ces OS supplémentaires lèvent significativement la complexité de l'OS dégénéré initial.
- Les séquence des OS 5', dont l'élongation par les polymérases d'ADN doit correspondre aux acides aminés situés en aval du motif peptidique considéré (motifs 1 et 2), sont celles du brin ADN (+), alors que celles des OS 3', à partir desquels l'élongation doit correspondre aux acides aminés situés en amont du motif peptidique considéré (motifs 2, 3 et 4), sont celles du brin ADN (-). Les OS correspondants au motif peptidique 2 ont été synthétisés sur les deux brins, pour pouvoir effectuer une élongation dans les deux sens.
- Chaque OS comprend des nucléotides supplémentaires permettant d'introduire en 5' la séquence correspondant à un site de restriction, EcoRI pour les OS5', BamHI pour les OS 3', et dans tous les cas une séquence GGAA 5'-terminale favorisant l'arrimage des polymérases et nucléases en amont du site de restriction.
- Suivant ces critères, les OS PTLVES' (83 a et b, 140 a à d) et PTLVE3' (145 a à d, 228 a à h, 241 a et b) (pour ***P**rimate **T-L**eukemia **V**irus-like **E**nv),* définis ci-dessus ont été synthétisés respectivement pour des élongations en 5' ou 3' du motif ciblé.

### 3. Mise au point des conditions d'amplification avec les oligonucléotidiques sur des séquences témoins

Pour la mise au point de l'amplification de séquences reconnues par les OS décrits ci-dessus, les inventeurs ont utilisé des préparations ADN témoins de plasmide contenant la séquence d'enveloppe HTLV-1 et des préparations témoins dépourvues de cette séquence. La stratégie d'amplification d'ADN qui a été sélectionnée consiste à enchaîner deux réactions d'amplification par un mélange des polymérases Taq et Pwo sur thermocycler dans les conditions dites de *"touch-down"* et combinant 2 couples d'OS différents.

Les premiers résultats d'amplification probants et reproductibles (amplification spécifiques de séquences HTLV sans amplification sur les préparations témoins) sont ceux obtenus avec la combinaison des OS PTLVE5'83b et PTLVE3'240b, pour la première réaction d'amplification, suivie d'une 2^{ème} réaction combinant les OS PTLVE5'83b et PTLVE3' 146a sur un échantillon de la 1^{ère} réaction. Dans les deux cas les conditions de *"touch-down"* incluent 15 cycles combinant chacun dénaturation à 94°C suivi d'une étape d'appariement et d'élongation effectuée à la même température, cette température étant comprise pour chaque cycle entre 65 et 50°C avec un pas décroissant de 1°C entre le 1" et le 15è cycle. Ces 15 cycles sont suivis de 30 cycles classiques d'amplification avec une température d'appariement à 50°C et d'élongation à 72°C.

### 4. Construction et séquençage d'une banque de fragments amplifiés à partir des réactions d'amplification

Un échantillon de la 2^{ème} réaction d'amplification décrite ci-dessus est utilisé pour générer une banque des séquences amplifiées. Pour cela 4 µl sur les 50 µl de la 2^{ème} réaction est utilisé pour ligation dans un vecteur de type pCR4-TOPO (Invitrogen) et transformation de bactéries. Entre 10 et 100 colonies résistantes à la kanamycine sont repiquées pour chaque ligation et mises en culture. L'ADN plasmidique de chaque colonie est analysé par séquençage en utilisant des séquences amorces universelles T3 et T7 du vecteur.

### II PREMIERS RESULTATS OBTENUS A PARTIR D'ECHANTILLONS HUMAINS ET DE PRIMATES

Les conditions décrites ci-dessus ont été appliquées à trois types d'échantillons d'ADN :
- Des échantillons d'ADN génomique de "patients séroindéterminés", caractérisés par une sérologie suggérant une infection antérieure par HTLV mais chez lesquels aucun diagnostic définitif n'a pu être établi. Chez ces patients, notamment, une recherche par amplification d'ADN de séquences HTLV *gag, pol* ou *tax* sont négatives.
- Des échantillons d'ADN génomique de "patients HTLV-1" chez lesquels une infection PTLV-1 caractéristique a été identifiée.
- Des échantillons d'ADN génomique de singes Mangabey agiles (*Cercocebus Agilis*) qui présentent une sérologie PTLV positive et chez lesquels des séquences Tax HTLV-1 ou STLV-L ont pu être amplifiées.

L'application de la méthode décrite ci-dessus a permis de détecter la présence de séquences de type SU de PTLV chez les trois types d'échantillons, y compris chez les "patients séroindéterminés".

L'analyse des séquences et de leurs capacités codantes au niveau de la région de SU concernée a permis de faire les observations suivantes:

### 1. Résultats obtenus sur "patients séroindéterminés"

En appliquant la méthode décrite ci-dessus sur l'ADN d'un "patient séroindéterminé" (échantillon No. 424), décrit comme ne portant pas de séquence de type HTLV, les inventeurs ont pourtant pu amplifier et caractériser des séquences de type SU de PTLV.

Au niveau nucléotidique, les séquences identifiées à partir de l'échantillon No. 424 sont de plusieurs types : des séquences HTLV-1 identiques à celles déjà décrites dans la littérature et de nouveaux variants. Au niveau codant, les séquences nucléotidiques se traduisent en trois types de séquences :
- Des séquences d'acides aminés identiques à celles des souches HTLV-1 déjà connues.
- Des variants de souches HTLV-1 avec 1 ou 2 résidus jamais décrits auparavant.
- Des variants de souches HTLV-1 avec 1 ou 2 résidus décrits comme communs aux seules souches HTLV-2 ou STLV-L.

### 2. Résultats obtenus sur "patients HTLV-1 typiques"

Au niveau nucléotidique, les séquences amplifiées à partir de l'échantillon provenant du "patient HTLV-1" (échantillon No.422) sont soit typiquement HTLV-1, telles que déjà décrites dans la littérature, soit des variants avec des répercussions sur la capacité codante. Au niveau codant, les séquences nucléotidiques se traduisent en trois types de séquences :
- Des séquences d'acides aminés identiques aux souches connues PTLV-1.
- Des variants HTLV-1 avec 1 ou 2 résidus typiques des HTLV-2, combinés ou pas avec des résidus jamais décrits auparavant.
- Des variants HTLV-2 combinant quelques résidus décrits pour être communs aux seules souches HTLV-2 ou STLV-L, ceci combinés ou pas avec des résidus jamais décrits auparavant.

### 3. Résultats obtenus sur singes Cercocebus Agilis

La méthode de l'invention a aussi permis d'amplifier des séquences de type SU de PTLV chez tous les Mangabey Agiles *(Cercocebus Agilis)* testés qui avaient été identifiés comme séropositifs pour PTLV. Au niveau nucléotidique, les séquences amplifiées à partir de ces singes sont soit celles des isolats déjà décrits auparavant, soit des variants nucléotidiques avec répercussions sur la capacité codante. Au niveau codant, les séquences nucléotidiques se traduisent en trois types de séquences :
- Des séquences d'acides aminés identiques aux souches connues HTLV-1.
- Des séquences d'acides aminés identiques à l'isolat STLV-3/CTO-604 tout récemment décrit chez un Mangabey à tête rouge (*Cercocebus Torquatus*) (Meertens et coll, 2002)
- Des séquences d'acides aminés du type STLV-3/CTO-604 avec 1 ou 2 résidus typiques des HTLV-2

### III. BIBLIOGRAPHIE

1. Battini, J. L., O. Danos, and J. M. Heard. 1995. Receptor-binding domain of murine leukemia virus envelope glycoproteins. J Virol. 69(2):713-719.
2.Battini, J. L., J. M. Heard, and O. Danos. 1992. Receptor choice determinants in the envelope glycoproteins of amphotropic, xenotropic, and polytropic murine leukemia viruses. J Virol. 66(3):1468-75.
3. Kim, F. J., I. Seiliez, C. Denesvre, D. Lavillette, F.-L. Cosset, and M. Sitbon. 2000. Definition of an amino-terminal domain of the human T-cell leukemia virus type 1 envelope surface unit that extends the fusogenic range of an ecotropic murine leukemia virus. J Biol Chem. 275(31):23417-20.
4**.**Lavillette, D., M. Maurice, C. Roche, S. J. Russell, M. Sitbon, and F. L. Cosset. 1998. A proline-rich motif downstream of the receptor binding domain modulates conformation and fusogenicity of murine retroviral envelopes. J Virol. 72(12):9955-65.
5.Lavillette, D., A. Ruggieri, S. J. Russell, and F. L. Cosset. 2000. Activation of a cell entry pathway common to type C mammalian retroviruses by soluble envelope fragments. J Virol. 74(1):295-304.
6.Meertens, L., R. Mahieux, P. Mauclere, J. Lewis, and A. Gessain. 2002. Complete Séquence of a Novel Highly Divergent Simian T-Cell Lymphotropic Virus from Wild-Caught Red-Capped Mangabeys (Cercocebus torquatus) from Cameroon: a New Primate T-Lymphotropic Virus Type 3 Subtype. J. Virol. 76(1):259-268.
7.Sitbon, M., L. d'Auriol, H. Ellerbrok, C. Andre, J. Nishio, S. Perryman, F. Pozo, S. F. Hayes, K. Wehrly, P. Tambourin, F. Galibert, and B. Chesebro. 1991. Substitution of leucine for isoleucine in a sequence highly conserved among retroviral envelope surface glycoproteins attenuates the lytic effect of the Friend murine leukemia virus. Proc Natl Acad Sci USA. 88(13):5932-6.

## Revendications

1. Utilisation de couples d'oligonucléotides dégénérés en orientation 5' et 3' comprenant 15 à 30 nucléotides, lesdits oligonucléotides dégénérés comprenant un mélange d'oligonucléotides codant pour les régions déterminées suivantes issues des fragments protéiques des protéines d'enveloppe des différentes souches des PTLV, telles que la protéine d'enveloppe de la souche MT-2 de HTLV-1, ou la souche NRA de HTLV-2, ou la souche de STLV-3, ou de séquences complémentaires aux séquences desdits oligonucléotides codant pour les régions déterminées suivantes :
| | |
|---|---|
| 83-YL/VFPHW-88 | SEQ ID NO : 1 |
| 140-NFTQ/REV-145 | SEQ ID NO : 2 |
| 222-NYS/TCI/MVC-228 | SEQ ID NO : 3 |
| 237-WHVLY-241 | SEQ ID NO : 4, |
et qui différent entre eux par substitution d'au moins un nucléotide par un autre de manière à ce que chaque oligonucléotide soit susceptible de coder pour la région déterminée susmentionnée,
pour la mise en oeuvre de procédés de détection de toute souche de PTLV (Primate T cell Lymphotrophic Virus), à savoir de toute souche appartenant aux HTLV-1, HTLV-2, STLV-1, STLV-2, et STLV-3, ainsi que de toute souche de virus apparentés aux PTLV, à savoir de toute souche dont la séquence en acides aminés déduite de la séquence nucléotidique codant pour la région aminoterminale de la composante de surface (SU) des protéines d'enveloppe présente un taux d'homologie d'au moins 30% avec les séquences en acides aminés codées par les séquences nucléotidiques correspondantes chez les PTLV, notamment pour la détection de nouveaux variants des PTLV, ou de virus comportant des séquences apparentées aux SU des PTLV,
lesdits procédés comprenant une étape d'amplification, à partir d'un échantillon biologique susceptible de contenir des PTLV, et à l'aide des oligonucléotides 5' et 3' dégénérés susmentionnés utilisés en tant qu'amorces, du nombre de copies de fragments nucléotidiques délimités en position 5' par l'oligonucléotide dégénéré en orientation 5', et en position 3' par l'oligonucléotide dégénéré en orientation 3', et une étape d'identification de la souche de PTLV contenue dans l'échantillon biologique à partir des fragments nucléotidiques amplifiés susmentionnés.

2. Utilisation selon la revendication 1, des oligonucléotides dégénérés en orientation 5' suivants :
- les oligonucléotides codant pour SEQ ID NO : 1 choisis parmi ceux de séquence SEQ
ID NO : 5 suivante :
| | |
|---|---|
| TAYBTNTTYCCNCAYTGG | SEQ ID NO : 5 |
dans laquelle :
Y représente C ou T,
B représente C, G ou T,
N représente A, C, G ou T,
telles que les amorces oligonucléotidiques 5' choisies parmi les suivantes :
| | | |
|---|---|---|
| PTLVE5'83a | TAYBTNTTYCCNCACTGG | SEQ ID NO : 6 |
| PTLVE5'83b | TAYBTNTTYCCNCATTGG | SEQ ID NO : 7 |
- les oligonucléotides codant pour SEQ ID NO : 2 choisis parmi ceux de séquence SEQ ID NO : 8 suivante :
| | |
|---|---|
| AAYTTYACNCARGARGT | SEQ ID NO : 8 |
dans laquelle :
Y représente C ou T,
R représente A ou G,
N représente A, C, G ou T,
telles que les amorces oligonucléotidiques 5' choisies parmi les suivantes :
| | | |
|---|---|---|
| PTLVE5'140a | AAYTTYACNCAAGAAGT | SEQ ID NO : 9 |
| PILVE5140b | AAYTTYACNCAGGAAGT | SEQ ID NO : 10 |
| PTLVE5'140c | AAYTTYACNCAAGAGGT | SEQ ID NO : 11 |
| PTLVE5'140d | AAYTTYACNCAGGAGGT | SEQ ID NO : 12 |

3. Utilisation selon la revendication 1 ou 2, d'oligonucléotides dégénérés en orientation 3' suivants :
- les oligonucléotides de séquences complémentaires aux séquences codant pour SEQ ID NO : 2 choisis parmi ceux de séquence SEQ ID NO : 13 suivante
| | |
|---|---|
| NACYTCYTGNGTRAARTT | SEQ ID NO : 13 |
dans laquelle :
Y représente C ou T,
R représente A ou G,
N représente A, C,G ou T,
telles que les amorces oligonucléotidiques 3' choisies parmi les suivantes :
| | | |
|---|---|---|
| PTLVE3'145a | NACYTCYTGNGTAAAATT | SEQ ID NO : 14 |
| PILVE3'145b | NACYTCYTGNGTGAAATT | SEQ ID NO : 15 |
| PTLVE3'145c | NACYTCYTGNGTAAAGTR | SEQ ID NO : 16 |
| PILVE3'145d | NACYTCYTGNGTGAAGTT | SEQ ID NO : 17 |
- les oligonucléotides de séquences complémentaires aux séquences codant pour SEQ ID NO : 3 choisis parmi ceux de séquence SEQ ID NO : 18 suivante :
| | |
|---|---|
| RMNACNATRCANSWRTARTT | SEQ ID NO : 18 |
dans laquelle :
R représente A ou G,
M représente A ou C,
S représente C ou G,
W représente A ou T,
N représente A, C, G ou T,
telles que les amorces oligonucléotidiques 3' choisies parmi les suivantes :
| | | |
|---|---|---|
| PTLVE3'228a | RMNACNATRCANSAATAATT | SEQ ID NO : 19 |
| PTLVE3'228b | RMNACNATRCANSAGTAATT | SEQ ID NO : 20 |
| PTLVE3'228c | RMNACNATRCANSAATAGTT | SEQ ID NO : 21 |
| PTLVE3'228d | RMNACNATRCANSAGTAGTT | SEQ ID NO : 22 |
| PTLVE3'228e | RMNACNATRCANSTATAATT | SEQ ID NO : 23 |
| PTLVE3'228f | RMNACNATRCANSTGTAATT | SEQ ID NO : 24 |
| PTLVE3'228g | RMNACNATRCANSTATAGTT | SEQ ID NO : 25 |
| PTLVE3'228h | RMNACNATRCANSTGTAGTT | SEQ ID NO : 26 |
- les oligonucléotides de séquences complémentaires aux séquences codant pour SEQ ID NO : 4 choisis parmi ceux de séquence SEQ ID NO : 27 suivante :
| | |
|---|---|
| RTANARNACRTGCCA | SEQ ID NO : 27 |
dans laquelle :
R représente A ou G,
N représente A, C, G ou T,
telles que les amorces oligonucléotidiques 3' choisies parmi les suivantes :
| | | |
|---|---|---|
| PTLVE3'241a | RTANARNACATGCCA | SEQ ID NO : 28 |
| PTLVE3'241b | RTANARNACGTGCCA | SEQ ID NO : 29 |

4. Utilisation selon l'une des revendications 1 à 3, **caractérisée en ce que** :
- les oligonucléotides dégénérés 5' sont choisis parmi :
* les oligonucléotides SEQ ID NO : 5 codant pour SEQ ID NO : 1,
* les oligonucléotides SEQ ID NO : 8 codant pour SEQ ID NO : 2,
- les oligonucléotides dégénérés 3' sont choisis parmi :
* les oligonucléotides SEQ ID NO : 13 de séquences complémentaires aux séquences codant pour SEQ ID NO : 2,
* les oligonucléotides SEQ ID NO : 18 de séquences complémentaires aux séquences codant pour SEQ ID NO : 3,
* les oligonucléotides SEQ ID NO : 27 de séquences complémentaires aux séquences codant pour SEQ ID NO : 4.

5. Utilisation selon l'une des revendications 1 à 4, où les oligonucléotides sont choisis de manière à ce qu'ils permettent l'amplification, à partir d'un échantillon biologique susceptible de contenir de l'ADN de PTLV, de séquences nucléotidiques codant pour des fragments protéiques comprenant une séquence délimitée du côté N-terminal par l'acide aminé situé en position 89, et du côté C-terminal par l'acide aminé situé en position 139 de la protéine d'enveloppe de la souche MT-2 de HTLV-1 représentée par SEQ ID NO : 43, ou comprenant une séquence analogue comprise dans la protéine d'enveloppe d'une autre souche de PTLV que HTLV-1, telle que la séquence délimitée du côté N-terminal par l'acide aminé situé en position 85, et du côté C-terminal par l'acide aminé situé en position 135 de la protéine d'enveloppe de la souche NRA de HTLV-2 représentée par SEQ ID NO : 45, ou la séquence délimitée du côté N-terminal par l'acide aminé situé en position 88, et du côté C-terminal par l'acide aminé situé en position 144 de la protéine d'enveloppe de la souche de STLV-3 représentée par SEQ ID NO : 47.

6. Utilisation selon la revendication 5, **caractérisée en ce que** :
- les oligonucléotides dégénérés 5' sont choisis parmi :
* les oligonucléotides SEQ ID NO : 5 codant pour SEQ ID NO : 1,
- les oligonucléotides dégénérés 3' sont choisis parmi :
* les oligonucléotides SEQ ID NO : 13 de séquences complémentaires aux séquences codant pour SEQ ID NO : 2,
* les oligonucléotides SEQ ID NO : 18 de séquences complémentaires aux séquences codant pour SEQ ID NO : 3,
* les oligonucléotides SEQ ID NO : 27 de séquences complémentaires aux séquences codant pour SEQ ID NO : 4.

7. Utilisation selon la revendication 5 ou 6, **caractérisée en ce que** :
- les oligonucléotides dégénérés 5' sont les suivants :
| | | |
|---|---|---|
| PTLVE5'83b | TAYBTNTTYCCNCATTGG | SEQ ID NO : 5 |
Y, B et N étant tels que définis dans la revendication 2,
- les oligonucléotides dégénérés 3' sont les suivants :
| | | |
|---|---|---|
| PTVE3'145a | NACYTCYTGNGTAAAATT | SEQ ID NO : 14 |
Y et N étant tels que définis dans la revendication 3.

8. Oligonucléotides tels que définis dans l'une des revendications 2 à 7, correspondants :
- aux oligonucléotides dégénérés en orientation 5' suivants :
* les oligonucléotides codant pour SEQ ID NO : 1 choisis parmi ceux de séquence SEQ ID NO : 5 suivante :
| | |
|---|---|
| TAYBTNTTYCCNCAYTGG | SEQ ID NO : 5 |
dans laquelle :
Y représente C ou T,
B représente C, G ou T,
N représente A, C, G ou T,
telles que les amorces oligonucléotidiques 5' choisies parmi les suivantes :
| | | |
|---|---|---|
| PTLVE5'83a | TAYBTNTTYCCNCACTGG | SEQ ID NO : 6 |
| PTLVE5'83b | TAYBTNTTYCCNCATTGG | SEQ ID NO : 7 |
- les oligonucléotides codant pour SEQ ID NO : 2 choisis parmi ceux de séquence SEQ
ID NO : 8 suivante :
| | |
|---|---|
| AAYTTYACNCARGARGT | SEQ ID NO : 8 |
dans laquelle :
Y représente C ou T,
R représente A ou G,
N représente A, C, G ou T,
telles que les amorces oligonucléotidiques 5' choisies parmi les suivantes :
| | | |
|---|---|---|
| PTVE5'140a | AAYTTYACNCAAGAAGT | SEQ ID NO : 9 |
| PTLVE5'140b | AAYTTYACNCAGGAAGT | SEQ ID NO : 10 |
| PTLVE5'140c | AAYTTYACNCAAGAGGT | SEQ ID NO : 11 |
| PTVE5'140d | AAYTTYACNCAGGAGGT | SEQ ID NO : 12 |
- aux oligonucléotides dégénérés en orientation 3' suivants :
* les oligonucléotides de séquences complémentaires aux séquences codant pour SEQ ID NO : 2 choisis parmi ceux de séquence SEQ ID NO : 13 suivante
| | |
|---|---|
| NACYTCYTGNGTRAARTT | SEQ ID NO : 13 |
dans laquelle :
Y représente C ou T,
R représente A ou G,
N représente A, C, G ou T,
telles que les amorces oligonucléotidiques 3' choisies parmi les suivantes :
| | | |
|---|---|---|
| PTLVE3'145a | NACYTCYTGNGTAAAATT | SEQ ID NO : 14 |
| PTLVE3'145b | NACYTCYTGNGTGAAATT | SEQ ID NO : 15 |
| PTLVE3'145c | NACYTCYTGNGTAAAGTT | SEQ ID NO : 16 |
| PTLVE3'145d | NACYTCYTGNGTGAAGTR | SEQ ID NO : 17 |
- les oligonucléotides de séquences complémentaires aux séquences codant pour SEQ ID NO : 3 choisis parmi ceux de séquence SEQ ID NO : 18 suivante :
| | |
|---|---|
| RMNACNATRCANSWRTARTT | SEQ ID NO : 18 |
dans laquelle :
R représente A ou G,
M représente A ou C,
S représente C ou G,
W représente A ou T,
N représente A, C, G ou T,
telles que les amorces oligonucléotidiques 3' choisies parmi les suivantes :
| | | |
|---|---|---|
| PTLVE3'228a | RMNACNATRCANSAATAATT | SEQ ID NO : 19 |
| PTLVE3'228b | RMNACNATRCANSAGTAATT | SEQ ID NO : 20 |
| PTLVE3'228c | RMNACNATRCANSAATAGTT | SEQ ID NO : 21 |
| PTLVE3'228d | RMNACNATRCANSAGTAGTT | SEQ ID NO : 22 |
| PTLVE3'228e | RMNACNATRCANSTATAATT | SEQ ID NO : 23 |
| PTLVE3'228f | RMNACNATRCANSTGTAATT | SEQ ID NO : 24 |
| PTLVE3'228g | RMNACNATRCANSTATAGTT | SEQ ID NO : 25 |
| PTLVE3'228h | RMNACNATRCANSTGTAGTT | SEQ ID NO : 26 |
- les oligonucléotides de séquences complémentaires aux séquences codant pour SEQ ID NO : 4 choisis parmi ceux de séquence SEQ ID NO : 27 suivante :
| | |
|---|---|
| RTANARNACRTGCCA | SEQ ID NO : 27 |
dans laquelle :
R représente A ou G,
N représente A, C, G ou T,
telles que les amorces oligonucléotidiques 3' choisies parmi les suivantes :
| | | |
|---|---|---|
| PTLVE3'241a | RTANARNACATGCCA | SEQ ID NO : 28 |
| PTLVE3'241b | RTANARNACGTGCCA | SEQ ID NO : 29 |

9. Procédé de détection de toute souche de PTLV, à savoir de toute souche appartenant aux HTLV-1, HTLV-2, STLV-1, STLV-2, et STLV-3, ainsi que de toute souche de virus comportant des séquences apparentées aux SU des PTLV, telles que définies dans la revendication 1, **caractérisé en ce qu'**il comprend :
- la mise en contact d'un couple d'oligonucléotides dégénérés 5' et 3' tels que définis dans l'une des revendications 1 à 7, avec l'ADN génomique ou l'ADN complémentaire dérivé à partir d'ARN extraits du contenu d'un échantillon biologique (tels que cellules sanguines, de moelle osseuse, biopsies, notamment de peau ou autres organes, ou frottis) susceptible de contenir des PTLV tels que définis ci-dessus,
- l'amplification de fragments d'ADN codant pour un fragment des protéines d'enveloppe des différentes souches des PTLV tel que défini dans la revendication 1 ou 5,
- la détection des fragments d'ADN amplifiés lors de l'étape précédente, cette détection pouvant être corrélée à la détection et le cas échéant à l'identification de PTLV tels que définis ci-dessus dans ledit échantillon biologique.

10. Procédé de détection de toute souche de PTLV selon la revendication 9, **caractérisé en ce que** l'étape d'amplification comprend la mise en oeuvre de deux réactions d'amplification, la deuxième réaction étant effectuée sur un échantillon de produits obtenus dans le cadre de la première réaction à l'aide des mêmes oligonucléotides 5' que dans le cas de la première réaction, et d'oligonucléotides 3' différents de ceux utilisés dans la première réaction, à savoir des amorces 3' dites « nichées » hybridant avec une région située plus en amont de la séquence codant pour la SU que les amorces 3' utilisées dans la première réaction.

11. Procédé de détection de toute souche de PTLV selon la revendication 9 ou 10, **caractérisé en ce qu'**il comprend :
- une première réaction d'amplification génique effectuée à l'aide de couples d'oligonucléotides dégénérés choisis parmi les couples :
* oligonucléotides SEQ ID NO : 5 / oligonucléotides SEQ ID NO : 18, ou
* oligonucléotides SEQ ID NO : 5 / oligonucléotides SEQ ID NO : 27, ou
* oligonucléotides SEQ ID NO : 8 / oligonucléotides SEQ ID NO : 27,
- et une deuxième étape d'amplification du nombre de copies de fragments d'ADN obtenus lors de l'étape précédente à l'aide de couples d'oligonucléotides dégénérés choisis respectivement parmi les couples :
* oligonucléotides SEQ ID NO : 5 / oligonucléotides SEQ ID NO : 13, ou
* oligonucléotides SEQ ID NO : 5 / oligonucléotides SEQ ID NO : 13 ou oligonucléotides SEQ ID NO : 18, ou
* oligonucléotides SEQ ID NO : 8 / oligonucléotides SEQ ID NO : 18,
- la détection des fragments d'ADN amplifiés lors de l'étape précédente, cette détection pouvant être corrélée à la détection et le cas échéant à l'identification de PTLV dans l'échantillon biologique.

12. Procédé de détection de toute souche de PTLV selon l'une des revendications 9 à 11, **caractérisé en ce qu'**il comprend :
- une première réaction d'amplification génique effectuée à l'aide de couples d'oligonucléotides dégénérés choisis de telle manière que :
* les oligonucléotides dégénérés 5' sont les suivants :
| | | |
|---|---|---|
| PTLVE5'83b | TAYBTNTTYCCNCATTGG | SEQ ID NO : 5 |
Y, B et N étant tels que définis dans la revendication 2,
* les oligonucléotides dégénérés 3' sont les suivants :
| | | |
|---|---|---|
| PTLVE3'241b | RTANARNACGTGCCA | SEQ ID NO : 29 |
R, et N étant tels que définis dans la revendication 3,
- une deuxième réaction d'amplification génique effectuée à l'aide de couples d'oligonucléotides dégénérés choisis de telle manière que :
* les oligonucléotides dégénérés 5' sont les suivants :
| | | |
|---|---|---|
| PTLVE5'83b | TAYBTNTTYCCNCATTGG | SEQ ID NO : 5 |
Y, B et N étant tels que définis dans la revendication 2,
* les oligonucléotides dégénérés 3' sont les suivants :
| | | |
|---|---|---|
| PTLVE3'145a | NACYTCYTGNGTAAAATT | SEQ ID NO : 14 |
Y et N étant tels que définis dans la revendication 3.

13. Trousse ou kit pour la mise en oeuvre d'un procédé de détection selon l'une des revendications 9 à 12, **caractérisé en ce qu'**il comprend un couple d'amorces tel que défini dans l'une des revendications 1 à 7 et, le cas échéant, les réactifs nécessaires à la mise en oeuvre de la réaction d'amplification par PCR et pour la détection des fragments amplifiés.

14. Application du procédé de détection selon l'une des revendications 9 à 12:
- au diagnostic *in vitro* de pathologies liées à une infection par un PTLV, ou par un virus comportant des séquences apparentées aux SU des PTLV, chez l'homme ou l'animal, telles que les hémopathies, les maladies auto-immunes, les maladies inflammatoires, les maladies dégénératives,
- au criblage et à l'identification de nouveaux agents infectieux chez l'homme ou l'animal, et plus particulièrement de nouvelles souches, ou variants, de virus susceptibles d'être classés dans les PTLV, ou de virus comportant des séquences apparentées aux SU des PTLV,
- au criblage de gènes de prédisposition ou de résistance à des pathologies chez l'homme
ou l'animal liées à la présence des PTLV ou de séquences apparentées, ou à une infection par un PTLV, telles que les hémopathies, les maladies auto-immunes, les maladies dégénératives,
- au criblage ou à la conception de nouveaux agents thérapeutiques comprenant des séquences entières ou partielles des protéines d'enveloppe de nouveaux variants de PTLV ainsi détectés,
- au criblage ou à la conception de nouveaux vecteurs de thérapie cellulaire utilisant les propriétés de tropisme des séquences entières ou partielles des protéines d'enveloppe de nouveaux variants de PTLV ainsi détectés.

## Claims

1. Use of pairs of degenerate oligonucleotides in 5' and 3' orientation comprising 15 to 30 nucleotides, said degenerate oligonucleotides comprising a mixture of oligonucleotides coding for the following determined regions corresponding to the protein fragments of the envelope proteins of the different strains of PTLVs, such as the envelope protein of the MT-2 strain of HTLV-1, or the NRA strain of HTLV-2, or the strain of STLV-3, or of the complementary sequences to said oligonucleotides coding for the following determined regions:
| | |
|---|---|
| 83-YL/VFPHW-88 | SEQ ID NO: 1 |
| 140-NFTQ/REV-145 | SEQ ID NO: 2 |
| 222-NYS/TCI/MVC-228 | SEQ ID NO: 3 |
| 237-WHVLY-241 | SEQ ID NO: 4 |
and which differ from each other by the substitution of at least one nucleotide by another in a manner such that each oligonucleotide is capable of coding for the abovementioned determined region,
for the implementation of processes for detecting any strain of PTLV (Primate T cell Lymphotrophic Virus), namely any strain belonging to HTLV-1, HTLV-2, STLV-1, STLV-2, and STLV-3, as well as any strain of virus belonging to PTLVs, namely any strain the amino acid sequence of which is deduced from the nucleotide sequence coding for the amino-terminal region of the surface component (SU) of the envelope proteins has a homology level of at least approximately 30% with the amino acid sequences coded by the corresponding nucleotide sequences in PTLVs, in particular for detecting novel variants of PTLVs, or a virus comprising sequences belonging to the SU of PTLVs,
said processes comprising an amplification stage, starting from a biological sample capable of containing PTLVs, and with the abovementioned degenerate 5' and 3' oligonucleotides used as primers, of the number of copies of nucleotide fragments delimited in position 5' by the degenerate oligonucleotide in 5'orientation, and in position 3' by the degenerate oligonucleotide in 3' orientation, and an identification stage of the strain of PTLV contained in the biological sample from the abovementioned amplified nucleotide fragments.

2. Use according to claim 1, of the following degenerated oligonucleotides in 5' orientation:
- the oligonucleotides coding for SEQ ID NO:1 chosen from those of the following SEQ ID NO: 5:
| | |
|---|---|
| TAYBTNTTYCCNCAYTGG | SEQ ID NO: 5 |
in which:
Y represents C or T,
B represents C, G or T,
N represents A, C, G or T,
such as the 5' oligonucleotide primers chosen from the following:
| | | |
|---|---|---|
| PTLVE5'83a | TAYBTNTTYCCNCACTGG | SEQ ID NO: 6 |
| PTLVE5'83b | TAYBTNTTYCCNCATTGG | SEQ ID NO: 7 |
- the oligonucleotides coding for SEQ ID NO: 2 chosen from those of the following SEQ ID NO: 8:
| | |
|---|---|
| AAYTTYACNCARGARGT | SEQ ID NO: 8 |
in which:
Y represents C or T,
R represents A or G,
N represents A, C, G or T,
such as the 5' oligonucleotide primers chosen from the following:
| | | |
|---|---|---|
| PTLVE5'140a | AAYTTYACNCAAGAAGT | SEQ ID NO: 9 |
| PTLVE5'140b | AAYTTYACNCAGGAAGT | SEQ ID NO: 10 |
| PTLVE5'140c | AAYTTYACNCAAGAGGT | SEQ ID NO: 11 |
| PTLVE5'140d | AAYTTYACNCAGGAGGT | SEQ ID NO: 12 |

3. Use according to claim 1 or 2, of the following degenerated oligonucleotides in 3' orientation:
- the oligonucleotides of complementary sequences to the sequences coding for SEQ ID NO: 2 chosen from those of the following SEQ ID NO: 13:
| | |
|---|---|
| NACYTCYTGNGTRAARTT | SEQ ID NO: 13 |
in which:
Y represents C or T,
R represents A or G,
N represents A, C, G or T,
such as the 3' oligonucleotide primers chosen from the following:
| | | |
|---|---|---|
| PTLVE3'145a | NACYTCYTGNGTAAAATT | SEQ ID NO: 14 |
| PTLVE3'145b | NACYTCYTGNGTGAAATT | SEQ ID NO: 15 |
| PTLVE3'145c | NACYTCYTGNGTAAAGTT | SEQ ID NO: 16 |
| PTLVE3'145d | NACYTCYTGNGTGAAGTT | SEQ ID NO: 17 |
- the oligonucleotides of complementary sequences to the sequences coding for SEQ ID NO: 3 chosen from those of the following SEQ ID NO: 18:
| | |
|---|---|
| RMNACNATRCANSWRTARTT | SEQ ID NO: 18 |
in which:
R represents A or G,
M represents A or C,
S represents C or G,
W represents A or T,
N represents A, C, G or T,
such as the 3' oligonucleotide primers chosen from the following:
| | | |
|---|---|---|
| PTLVE3'228a | RMNACNATRCANSAATAATT | SEQ ID NO: 19 |
| PTLVE3'228b | RMNACNATRCANSAGTAATT | SEQ ID NO: 20 |
| PTLVE3'228c | RMNACNATRCANSAATAGTT | SEQ ID NO: 21 |
| PTLVE3'228d | RMNACNATRCANSAGTAGTT | SEQ ID NO: 22 |
| PTLVE3'228e | RMNACNATRCANSTATAATT | SEQ ID NO: 23 |
| PTLVE3'228f | RMNACNATRCANSTGTAATT | SEQ ID NO: 24 |
| PTLVE3'228g | RMNACNATRCANSTATAGTT | SEQ ID NO: 25 |
| PTLVE3'228h | RMNACNATRCANSTGTAGTT | SEQ ID NO: 26 |
- the oligonucleotides of complementary sequences to the sequences coding for SEQ ID NO: 4 chosen from those of the following SEQ ID NO: 27:
| | |
|---|---|
| RTANARNACRTGCCA | SEQ ID NO: 27 |
in which:
R represents A or G,
N represents A, C, G or T,
such as the 3' oligonucleotide primers chosen from the following:
| | | |
|---|---|---|
| PTLVE3'241a | RTANARNACATGCCA | SEQ ID NO: 28 |
| PTLVE3'241b | RTANARNACGTGCCA | SEQ ID NO: 29 |

4. Use according to one of claims 1 to 3, **characterized in that**:
- the degenerate 5' oligonucleotides are chosen among:
* the oligonucleotides of SEQ ID NO: 5 coding for SEQ ID NO: 1,
* the oligonucleotides of SEQ ID NO: 8 coding for SEQ ID NO: 2,
- the degenerate 3' oligonucleotides are chosen among:
* the oligonucleotides of SEQ ID NO: 13 having complementary sequences to the sequences coding for SEQ ID NO: 2,
* the oligonucleotides of SEQ ID NO: 18 having complementary sequences to the sequences coding for SEQ ID NO: 3,
* the oligonucleotides of SEQ ID NO: 27 having complementary sequences to the sequences coding for SEQ ID NO: 4,

5. Use according to one of claims 1 to 4, said oligonucleotides being chosen in such a way that they allow the amplification, starting from a biological sample capable of containing the DNA of PTLV, of nucleotide sequences coding for the protein fragments comprising a sequence delimited on the N-terminal side by the amino acid situated in position 89, and on the C-terminal side by the amino acid situated in position 139 of the envelope protein of the MT-2 strain of HTLV-1 represented by SEQ ID NO: 43, or comprising an analogous sequence comprised in the envelope protein of a strain of PTLV other than PTLV-1, such as the sequence delimited on the N-terminal side by the amino acid situated in position 85, and on the C-terminal side by the amino acid situated in position 135 of the envelope protein of the NRA strain of HTLV-2 represented by SEQ ID NO: 45, or the sequence delimited on the N-terminal side by the amino acid situated in position 88, and on the C-terminal side by the amino acid situated in position 144 of the envelope protein of the strain of STLV-3 represented by SEQ ID NO: 47.

6. Use according to claim 5, **characterized in that**:
- the degenerate 5' oligonucleotides are chosen among:
* the oligonucleotides of SEQ ID NO: 5 coding for SEQ ID NO: 1,
- the degenerate 3' oligonucleotides are chosen among:
* the oligonucleotides of SEQ ID NO: 13 having complementary sequences to the sequences coding for SEQ ID NO: 2,
* the oligonucleotides of SEQ ID NO: 18 having complementary sequences to the sequences coding for SEQ ID NO: 3,
* the oligonucleotides of SEQ ID NO: 27 having complementary sequences to the sequences coding for SEQ ID NO: 4,

7. Use according to claim 5 or 6, **characterized in that**:
- the degenerate 5' oligonucleotides are the following:
| | | |
|---|---|---|
| PTLVE5'83b | TAYBTNTTYCCNCATTGG | SEQ ID NO: 5 |
Y, B and N being as defined in claim 2,
- the degenerate 3' oligonucleotides are the following:
| | | |
|---|---|---|
| PTLVE3'145a | NACYTCYTGNGTAAAATT | SEQ ID NO: 14 |
Y and N being as defined in claim 3.

8. Oligonucleotides according to one of claims 2 to 7, corresponding:
- to the following degenerate oligonucleotides in 5' orientation:
* the oligonucleotides coding for SEQ ID NO:1 chosen from those of the following SEQ ID NO: 5:
| | |
|---|---|
| TAYBTNTTYCCNCAYTGG | SEQ ID NO: 5 |
in which:
Y represents C or T,
B represents C, G or T,
N represents A, C, G or T,
such as the 5' oligonucleotide primers chosen from the following:
| | | |
|---|---|---|
| PTLVE5'83a | TAYBTNTTYCCNCACTGG | SEQ ID NO: 6 |
| PTLVE5'83b | TAYBTNTTYCCNCATTGG | SEQ ID NO: 7 |
* the oligonucleotides coding for SEQ ID NO: 2 chosen from those of the following SEQ ID NO: 8:
| | |
|---|---|
| AAYTTYACNCARGARGT | SEQ ID NO: 8 |
in which:
Y represents C or T,
R represents A or G,
N represents A, C, G or T,
such as the 5' oligonucleotide primers chosen from the following:
| | | |
|---|---|---|
| PTLVE5'140a | AAYTTYACNCAAGAAGT | SEQ ID NO: 9 |
| PTLVE5'140b | AAYTTYACNCAGGAAGT | SEQ ID NO: 10 |
| PTLVE5'140c | AAYTTYACNCAAGAGGT | SEQ ID NO: 11 |
| PTLVE5' 140d | AAYTTYACNCAGGAGGT | SEQ ID NO: 12 |
- to the degenerate oligonucleotides in 3' orientation:
* the oligonucleotides of complementary sequences to the sequences coding for SEQ ID NO: 2 chosen from those of the following SEQ ID NO: 13:
| | |
|---|---|
| NACYTCYTGNGTRAARTT | SEQ ID NO: 13 |
in which:
Y represents C or T,
R represents A or G,
N represents A, C, G or T,
such as the 3' oligonucleotide primers chosen from the following:
| | | |
|---|---|---|
| PTLVE3'145a | NACYTCYTGNGTAAAATT | SEQ ID NO: 14 |
| PTLVE3'145b | NACYTCYTGNGTGAAATT | SEQ ID NO: 15 |
| PTLVE3'145c | NACYTCYTGNGTAAAGTT | SEQ ID NO: 16 |
| PTLVE3'145d | NACYTCYTGNGTGAAGTT | SEQ ID NO: 17 |
* the oligonucleotides of complementary sequences to the sequences coding for SEQ ID NO: 3 chosen from those of the following SEQ ID NO: 18:
| | |
|---|---|
| RMNACNATRCANSWRTARTT | SEQ ID NO: 18 |
in which:
R represents A or G,
M represents A or C,
S represents C or G,
W represents A or T,
N represents A, C, G or T,
such as the 3' oligonucleotide primers chosen from the following:
| | | |
|---|---|---|
| PTLVE3'228a | RMNACNATRCANSAATAATT | SEQ ID NO: 19 |
| PTLVE3'228b | RMNACNATRCANSAGTAATT | SEQ ID NO: 20 |
| PTLVE3'228c | RMNACNATRCANSAATAGTT | SEQ ID NO: 21 |
| PTLVE3'228d | RMNACNATRCANSAGTAGTT | SEQ ID NO: 22 |
| PTLVE3'228e | RMNACNATRCANSTATAATT | SEQ ID NO: 23 |
| PTLVE3'228f | RMNACNATRCANSTGTAATT | SEQ ID NO: 24 |
| PTLVE3'228g | RMNACNATRCANSTATAGTT | SEQ ID NO: 25 |
| PTLVE3'228h | RMNACNATRCANSTGTAGTT | SEQ ID NO: 26 |
* the oligonucleotides of complementary sequences to the sequences coding for SEQ ID NO: 4 chosen from those of the following SEQ ID NO: 27:
| | |
|---|---|
| RTANARNACRTGCCA | SEQ ID NO: 27 |
in which:
R represents A or G,
N represents A, C, G or T,
such as the 3' oligonucleotide primers chosen from the following:
| | | |
|---|---|---|
| PTLVE3'241a | RTANARNACATGCCA | SEQ ID NO: 28 |
| PTLVE3'241b | RTANARNACGTGCCA | SEQ ID NO: 29 |

9. Process for detecting any strain of PTLV, namely of any strain belonging to HTLV-1, HTLV-2, STLV-1, STLV-2, and STLV-3, as well as any strain of virus comprising the sequences belonging to the SUs of PTLVs, as defined in claim 1, **characterized in that** it comprises:
- the bringing into contact of a pair of degenerate 5' and 3' oligonucleotides as defined in one of claims 1 to 7, with the genomic DNA or complementary DNA derived from RNA extracts of the content of a biological sample (such as blood cells, bone marrow, biopsies, in particular of the skin or other organs, or smears) capable of containing PTLVs as defined above,
- the amplification of DNA fragments coding for a fragment of the envelope proteins of the different strains of PTLVs as defined in claim 1 or 5,
- the detection of the DNA fragments amplified during the previous stage, this detection being able to be correlated to the detection and if appropriate to the identification of PTLV as defined above in said biological sample.

10. Process for detecting any strain of PTLV according to claim 9, **characterized in that** the amplification stage comprises the implementation of two amplification reactions, the second reaction being carried out on a sample of products obtained within the context of the first reaction using the same 5' oligonucleotides as in the case of the first reaction, and 3' oligonucleotides which are different from those used in the first reaction, namely the so-called "nested" 3' primers hybridizing with a region situated more upstream of the sequence coding for the SU than the 3' primers used in the first reaction.

11. Process for detecting any strain of PTLV according to claim 9 or 10, **characterized in that** it comprises:
- a first gene amplification reaction carried out using pairs of degenerate oligonucleotides chosen from the pairs:
* oligonucleotides of SEQ ID NO: 5/oligonucleotides of SEQ ID NO: 18 or
* oligonucleotides of SEQ ID NO: 5/oligonucleotides of SEQ ID NO: 27 or
* oligonucleotides of SEQ ID NO: 8/oligonucleotides of SEQ ID NO: 27,
- and a second amplification stage of the number of copies of DNA fragments obtained during the previous stage using pairs of degenerate oligonucleotides chosen respectively from the pairs:
* oligonucleotides of SEQ ID NO: 5/oligonucleotides of SEQ ID NO: 13, or
* oligonucleotides of SEQ ID NO: 5/oligonucleotides of SEQ ID NO: 13, or oligonucleotides of SEQ ID NO: 18, or
* oligonucleotides of SEQ ID NO: 8/oligonucleotides of SEQ ID NO: 18,
- the detection of the DNA fragments amplified during the previous stage, this detection being able to be correlated to the detection and if appropriate to the identification of PTLV in the biological sample.

12. Process for detecting any strain of PTLV according to one of claims 9 to 11, **characterized in that** it comprises:
- a first gene amplification reaction carried out using pairs of degenerate oligonucleotides chosen in such a way that :
* the degenerate 5' oligonucleotides are the following:
| | | |
|---|---|---|
| PTLVE5'83b | TAYBTNTTYCCNCATTGG | SEQ ID NO: 5 |
Y, B and N being as defined in claim 2,
* the degenerate 3' oligonucleotides are the following:
| | | |
|---|---|---|
| PTLVE3'241b | RTANARNACGTGCCA | SEQ ID NO: 29 |
R, and N being as defined in claim 3,
- a second gene amplification reaction carried out using pairs of degenerate oligonucleotides chosen in such a way that:
* the degenerate 5' oligonucleotides are the following:
| | | |
|---|---|---|
| PTLVES'83b | TAYBTNTTYCCNCATTGG | SEQ ID NO: 5 |
Y, B and N being as defined in claim 2,
* the degenerate 3' oligonucleotides are the following:
| | | |
|---|---|---|
| PTLVE3'145a | NACYTCYTGNGTAAAATT | SEQ ID NO: 14 |
Y and N being as defined in claim 3.

13. Kit for the implementation of a detection process according to one of claims 9 to 12, **characterized in that** it comprises a pair of primers as defined in one of claims 1 to 7, and, if appropriate, the reagents necessary for the implementation of the amplification reaction by PCR and for the detection of the amplified fragments.

14. Application of the detection process according to one of claims 9 to 12:
- to the *in vitro* diagnosis of pathologies linked to an infection by a PTLV, or by a virus comprising the sequences belonging to the SU of PTLVs, in man or animals, such as hemopathies, autoimmune diseases, inflammatory diseases, degenerative diseases,
- to the screening and identification of novel infectious agents in man or animals, and more particularly novel strains, or variants, of a virus which can be classed in PTLVs, or a virus comprising the sequences belonging to the SU of PTLVs,
- to the screening of genes with a predisposition or a resistance to the pathologies in man or animals linked to the presence of PTLVs or of related sequences, or to an infection by a PTLV, such as hemopathies, autoimmune diseases, degenerative diseases,
- to the screening or the design of novel therapeutic agents comprising the entire or partial sequences of the envelope proteins of novel variants of PTLV thus detected,
- to the screening or the design of novel cell therapy vectors using the tropism proprieties of the entire or partial sequences of the envelope proteins of novel variants of PTLV thus detected.

## Patentansprüche

1. Verwendung von Paaren degenerierter Oligonukleotide in 5'- und 3'-Richtung, die 15 bis 30 Nukleotide umfassen, wobei die degenerierten Oligonukleotide ein Gemisch aus Oligonukleotiden umfassen, das für die folgenden determinierten Regionen kodiert, die aus Proteinfragmenten von Hüllproteinen der verschiedenen Stämme der PTLV stammen, wie etwa dem Hüllprotein des MT-2-Stamms des HTLV-1, oder des NRA-Stamms des HTLV-2, oder des Stamms des STLV-3, oder der Sequenzen, die komplementär zu den Sequenzen der Oligonukleotide sind, die für die folgenden determinierten Regionen kodieren:
| | |
|---|---|
| 83-YL/VFPHW-88 | SEQ ID Nr. 1 |
| 140-NFTQ/REV-145 | SEQ ID Nr. 2 |
| 222-NYS/TCI/MVC-228 | SEQ ID Nr. 3 |
| 237-WHVLY-241 | SEQ ID Nr. 4, |
und die sich voneinander durch Substitution mindestens eines Nukleotids durch ein anderes unterscheiden, derart, dass jedes Oligonukleotid für die oben genannte determinierte Region kodieren kann,
zum Durchführen von Verfahren zum Nachweis jedes Stamms des PTLV (Primate T cell Lymphotrophic Virus), nämlich jedes Stamms, der zu HTLV-1, HTLV-2, STLV-1, STLV-2 und STLV-3 gehört, sowie jedes Virenstamms, der mit den PTLV verwandt ist, nämlich jedes Stamms, dessen Aminosäuresequenz, die von der Nukleinsäuresequenz abgeleitet ist, die für die aminoterminale Region der Oberflächenkomponente (SU) der Hüllproteine kodiert, einen Homologiegrad von mindestens etwa 30 % mit den Aminosäuresequenzen aufweist, die von den entsprechenden Nukleotidsequenzen des PTLV kodiert werden, vor allem zum Nachweis neuer Varianten der PTLV, oder von Viren, die Sequenzen umfassen, die mit SU der PTLV verwandt sind,
wobei die Verfahren einen Schritt der Amplifikation, ausgehend von einer biologischen Probe, die PTLV enthalten kann, und mit Hilfe der oben genannten degenerierten 5'- und 3'-Oligonukleotide, die als Primer verwendet werden, der Kopienanzahl der Nukleotidfragmente, die an Position 5' durch das degenerierte Oligonukleotid in 5'-Richtung, und an Position 3' durch das degenerierte Oligonukleotid in 3'-Richtung begrenzt sind, und einen Schritt der Identifikation des PTLV-Stamms umfassen, der in der biologischen Probe enthalten ist, aus den oben genannten amplifizierten Nukleotidfragmenten.

2. Verwendung nach Anspruch 1 der folgenden degenerierten Oligonukleotide in 5'-Richtung:
- Oligonukleotide, die für SEQ ID Nr. 1 kodieren, ausgewählt aus jenen, mit der folgenden Sequenz SEQ ID Nr. 5:
| | |
|---|---|
| TAYBTNTTYCCNCAYTGG | SEQ ID Nr. 5 |
wobei:
Y für C oder T steht,
B für C, G oder T steht,
N für A, C, G oder T steht,
wie etwa die 5'-Oligonukleotidprimer, ausgewählt aus den Folgenden:
| | | |
|---|---|---|
| PTLVE5'83a | TAYBTNTTYCCNCACTGG | SEQ ID Nr. 6 |
| PTLVE5'83b | TAYBTNTTYCCNCATTGG | SEQ ID Nr. 7 |
- Oligonukleotide, die für SEQ ID Nr. 2 kodieren, ausgewählt aus jenen, mit der folgenden Sequenz SEQ ID Nr. 8:
| | |
|---|---|
| AAYTTYACNCARGARGT | SEQ ID Nr. 8 |
wobei:
Y für C oder T steht,
R für A oder G steht,
N für A, C, G oder T steht,
wie etwa die 5'-Oligonukleotidprimer, ausgewählt aus den Folgenden:
| | | |
|---|---|---|
| PTLVE5'140a | AAYTTYACNCAAGAAGT | SEQ ID Nr. 9 |
| PTLVE5'140b | AAYTTYACNCAGGAAGT | SEQ ID Nr. 10 |
| PTLVE5'140c | AAYTTYACNCAAGAGGT | SEQ ID Nr. 11 |
| PTLVE5'140d | AAYITYACNCAGGAGGT | SEQ ID Nr. 12. |

3. Verwendung nach einem der Ansprüche 1 oder 2 der folgenden degenerierten Oligonukleotide in 3'-Richtung:
- Oligonukleotide der Sequenzen, die komplementär zu den Sequenzen sind, die für SEQ ID Nr. 2 kodieren, ausgewählt aus jenen, mit der folgenden Sequenz SEQ ID Nr. 13:
| | |
|---|---|
| NACYTCYTGNGTRAARTT | SEQ ID Nr. 13 |
wobei:
Y für C oder T steht,
R für A oder G steht,
N für A, C, G oder T steht,
wie etwa die 3'-Oligonukleotidprimer, ausgewählt aus den Folgenden:
| | | |
|---|---|---|
| PTLVE3'145a | NACYTCYTGNGTAAAATT | SEQ ID Nr. 14 |
| PTLVE3'145b | NACYTCYTGNGTGAAATT | SEQ ID Nr. 15 |
| PTLVE3'145c | NACYTCYTGNGTAAAGTT | SEQ ID Nr. 16 |
| PTLVE3'145d | NACYTCYTGNGTGAAGT | SEQ ID Nr. 17, |
- Oligonukleotide der Sequenzen, die komplementär zu den Sequenzen sind, die für SEQ ID Nr. 3 kodieren, ausgewählt aus jenen, mit der folgenden Sequenz SEQ ID Nr. 18:
| | |
|---|---|
| RMNACNATRCANSWRTARTT | SEQ ID Nr. 18 |
wobei:
R für A oder G steht,
M für A oder C steht,
S für C oder G steht,
W für A oder T steht,
N für A, C, G oder T steht,
wie etwa die 3'-Oligonukleotidprimer, ausgewählt aus den Folgenden:
| | | |
|---|---|---|
| PTLVE3'228a | RMNACNATRCANSAATAATT | SEQ ID Nr. 19 |
| PTLVE3'228b | RMNACNATRCANSAGTAATT | SEQ ID Nr. 20 |
| PTLVE3'228c | RMNACNATRCANSAATAGTT | SEQ ID Nr. 21 |
| PTLVE3'228d | RMNACNATRCANSAGTAGTT | SEQ ID Nr. 22 |
| PTLVE3'228e | RMNACNATRCANSTATAATT | SEQ ID Nr. 23 |
| PTLVE3'228f | RMNACNATRCANSTGTAATT | SEQ ID Nr. 24 |
| PTLVE3'228g | RMNACNATRCANSTATAGTT | SEQ ID Nr. 25 |
| PTLVE3'228h | RMNACNATRCANSTGTAGTT | SEQ ID Nr. 26 |
- Oligonukleotide der Sequenzen, die komplementär zu den Sequenzen sind, die für SEQ ID Nr. 4 kodieren, ausgewählt aus jenen, mit der folgenden Sequenz SEQ ID Nr. 27:
| | |
|---|---|
| RTANARNACRTGCCA | SEQ ID Nr. 27 |
wobei:
R für A oder G steht,
N für A, C, G oder T steht,
wie etwa die 3'-Oligonukleotidprimer, ausgewählt aus den Folgenden:
| | | |
|---|---|---|
| PTLVE3'241a | RTANARNACATGCCA | SEQ ID Nr. 28 |
| PTLVE3'241b | RTANARNACGTGCCA | SEQ ID Nr. 29 |

4. Verwendung nach einem der Ansprüche 1 bis 3, **dadurch gekennzeichnet, dass**:
- die degenerierten Oligonukleotide 5' ausgewählt sind aus:
* den Oligonukleotiden mit SEQ ID Nr. 5, die für SEQ ID Nr. 1 kodieren,
* den Oligonukleotiden mit SEQ ID Nr. 8, die für SEQ ID Nr. 2 kodieren,
- die degenerierten Oligonukleotide 3' ausgewählt sind aus:
* den Oligonukleotiden mit SEQ ID Nr. 13 der Sequenzen, die komplementär sind zu den Sequenzen, die für SEQ ID Nr. 2 kodieren,
* den Oligonukleotiden mit SEQ ID Nr. 18 der Sequenzen, die komplementär sind zu den Sequenzen, die für SEQ ID Nr. 3 kodieren,
* den Oligonukleotiden mit SEQ ID Nr. 27 der Sequenzen, die komplementär sind zu den Sequenzen, die für SEQ ID Nr. 4 kodieren.

5. Verwendung nach einem der Ansprüche 1 bis 4, wobei die Oligonukleotide derart ausgewählt sind, dass sie die Amplifikation, ausgehend von einer biologischen Probe, die DNA von PTLV enthalten kann, von Nukleotidsequenzen ermöglichen, die für Proteinfragmente kodieren, die eine Sequenz umfassen, die an der N-terminalen Seite durch die Aminosäure begrenzt ist, die an Position 89 liegt, und an der C-terminalen Seiten durch die Aminosäure, die an Position 139 liegt, des Hüllproteins des MT-2-Stamms von HTLV-1, für die die SEQ ID Nr. 43 steht, oder eine analoge Sequenz umfasst, die im Hüllprotein eines anderen PTLV-Stamms als HTLV-1 enthalten ist, wie etwa die Sequenz, die an der N-terminalen Seite durch die Aminosäure begrenzt ist, die an Position 85 liegt, und an der C-terminalen Seite durch die Aminosäure, die an Position 135 des Hüllproteins liegt, des NRA-Stamms von HTLV-2, für die die SEQ ID Nr. 45 steht, oder die Sequenz, die an der N-terminalen Seite durch die Aminosäure begrenzt ist, die an Position 88 liegt, und an der C-terminalen Seite durch die Aminosäure, die an Position 144 liegt, des Hüllproteins des STLV-3-Stamms, für die die SEQ ID Nr. 47 steht.

6. Verwendung nach Anspruch 5, **dadurch gekennzeichnet, dass**:
- die degenerierten Oligonukleotide 5' ausgewählt sind aus:
* den Oligonukleotiden mit SEQ ID Nr. 5, die für SEQ ID Nr. 1 kodieren,
- die degenerierten Oligonukleotide 3' ausgewählt sind aus:
* den Oligonukleotiden mit SEQ ID Nr. 13, der Sequenzen, die komplementär sind zu den Sequenzen, die für SEQ ID Nr. 2 kodieren,
* den Oligonukleotiden mit SEQ ID Nr. 18, der Sequenzen, die komplementär sind zu den Sequenzen, die für SEQ ID Nr. 3 kodieren,
* den Oligonukleotiden mit SEQ ID Nr. 27 der Sequenzen, die komplementär sind zu den Sequenzen, die für SEQ ID Nr. 4 kodieren.

7. Verwendung nach Anspruch 5 oder 6, **dadurch gekennzeichnet, dass**:
- die degenerierten Oligonukleotide 5' Folgende sind:
| | | |
|---|---|---|
| PTLVE5'83b | TAYBTNTTYCCNCATTGG | SEQ ID Nr. 5 |
wobei Y, B und N sind, wie in Anspruch 2 definiert,
- die degenerierten Oligonukleotide 3' Folgende sind:
| | | |
|---|---|---|
| PTLVE3'145a | NACYTCYTGNGTAAAATT | SEQ ID Nr. 14 |
wobei Y und N sind, wie in Anspruch 3 definiert,

8. Oligonukleotide, wie in einem der Ansprüche 2 bis 7 definiert, die:
- den folgenden degenerierten Oligonukleotiden in 5'-Richtung entsprechen:
* Oligonukleotiden, die für SEQ ID Nr. 1 kodieren, ausgewählt aus jenen, mit der folgenden Sequenz SEQ ID Nr. 5:
| | |
|---|---|
| TAYBTNTTYCCNCAYTGG | SEQ ID Nr. 5 |
wobei:
Y für C oder T steht, B für C, G oder T steht, N für A, C, G oder T steht,
wie etwa die 5'-Oligonukleotidprimer, ausgewählt aus den Folgenden:
| | | |
|---|---|---|
| PTLVE5'83a | TAYBTNTTYCCNCACTGG | SEQ ID Nr. 6 |
| PTLVE5'83b | TAYBTNTTYCCNCATTGG | SEQ ID Nr. 7 |
Oligonukleotiden, die für SEQ ID Nr. 2 kodieren, ausgewählt aus jenen, mit der folgenden Sequenz SEQ ID Nr. 8:
| | |
|---|---|
| AAYTTYACNCARGARGT | SEQ ID Nr. 8 |
wobei:
Y für C oder T steht,
R für A oder G steht,
N für A, C, G oder T steht,
wie etwa die 5'-Oligonukleotidprimer, ausgewählt aus den Folgenden:
| | | |
|---|---|---|
| PTLVE5'140a | AAYTTYACNCAAGAAGT | SEQ ID Nr. 9 |
| PTLVE5'140b | AAYTTYACNCAGGAAGT | SEQ ID Nr. 10 |
| PTLVE5'140c | AAYTTYACNCAAGAGGT | SEQ ID Nr. 11 |
| PTLVE5'140d | AAYITYACNCAGGAGGT | SEQ ID Nr. 12 |
- den folgenden degenerierten Oligonukleotiden in 3'-Richtung:
* Oligonukleotide der Sequenzen, die komplementär zu den Sequenzen sind, die für SEQ ID Nr. 2 kodieren, ausgewählt aus jenen, mit der folgenden Sequenz SEQ ID Nr. 13:
| | |
|---|---|
| NACYTCYTGNGTRAARTT | SEQ ID Nr. 13 |
wobei:
Y für C oder T steht,
R für A oder G steht,
N für A, C, G oder T steht,
wie etwa die 3'-Oligonukleotidprimer, ausgewählt aus den Folgenden:
| | | |
|---|---|---|
| PTLVE3'145a | NACYTCYTGNGTAAAATT | SEQ ID Nr. 14 |
| PTLVE3'145b | NACYTCYTGNGTGAAATT | SEQ ID Nr. 15 |
| PTLVE3'145c | NACYTCYTGNGTAAAGTT | SEQ ID Nr. 16 |
| PTLVE3'145d | NACYTCYTGNGTGAAGTT | SEQ ID Nr. 17 |
- Oligonukleotide der Sequenzen, die komplementär zu den Sequenzen sind, die für SEQ ID Nr. 3 kodieren, ausgewählt aus jenen, mit der folgenden Sequenz SEQ ID Nr. 18:
| | |
|---|---|
| RMNACNATRCANSWRTARTT | SEQ ID Nr. 18 |
wobei:
R für A oder G steht,
M für A oder C steht,
S für C oder G steht,
W für A oder T steht,
N für A, C, G oder T steht,
wie etwa die 3'-Oligonukleotidprimer, ausgewählt aus den Folgenden:
| | | |
|---|---|---|
| PTLVE3'228a | RMNACNATRCANSAATAATT | SEQ ID Nr. 19 |
| PTLVE3'228b | RMNACNATRCANSAGTAATT | SEQ ID Nr. 20 |
| PTLVE3'228c | RMNACNATRCANSAATAGTT | SEQ ID Nr. 21 |
| PTLVE3'228d | RMNACNATRCANSAGTAGTT | SEQ ID Nr. 22 |
| PTLVE3'228e | RMNACNATRCANSTATAATT | SEQ ID Nr. 23 |
| PTLVE3'228f | RMNACNATRCANSTGTAATT | SEQ ID Nr. 24 |
| PTLVE3'228g | RMNACNATRCANSTATAGTT | SEQ ID Nr. 25 |
| PTLVE3'228h | RMNACNATRCANSTGTAGTT | SEQ ID Nr. 26, |
- Oligonukleotide der Sequenzen, die komplementär zu den Sequenzen sind, die für SEQ ID Nr. 4 kodieren, ausgewählt aus jenen, mit der folgenden Sequenz SEQ ID Nr. 27:
| | |
|---|---|
| RTANARNACRTGCCA | SEQ ID Nr. 27 |
wobei:
R für A oder G steht,
N für A, C, G oder T steht,
wie etwa die 3'-Oligonukleotidprimer, ausgewählt aus den Folgenden:
| | | |
|---|---|---|
| PTLVE3'241a | RTANARNACATGCCA | SEQ ID Nr. 28 |
| PTLVE3'241b | RTANARNACGTGCCA | SEQ ID Nr. 29. |

9. Verfahren zum Nachweis jedes PTLV-Stamms, nämlich jedes Stamms, der zu HTLV-1, HTLV-2, STLV-1, STLV-2 und STLV-3 gehört, sowie jedes Virenstamms, der Sequenzen umfasst, die mit der SU der PTLV verwandt sind, wie in Anspruch 1 definiert, **dadurch gekennzeichnet, dass** es umfasst:
- das Inkontaktbringen eines Paars von degenerierten Oligonukleotiden 5' und 3', wie in einem der Ansprüche 1 bis 7 definiert, mit genomischer DNA oder der komplementären DNA, abgeleitet ausgehend von RNA, die aus dem Inhalt einer biologischen Probe extrahiert wurde (wie etwa Blutzellen, Knochenmark, Biopsien, insbesondere der Haut oder anderer Organe, oder Abstriche), die PTLV enthalten können, wie oben definiert,
- die Amplifikation von DNA-Fragmenten, die für ein Fragment der Hüllproteine der verschiedenen Stämme der PTLV kodieren, wie in Anspruch 1 oder 5 definiert,
- der Nachweis von DNA-Fragmenten, die im vorhergehenden Schritt amplifiziert wurden, wobei dieser Nachweis mit dem Nachweis, und gegebenenfalls der Identifikation von PTLV, wie oben definiert, in der biologischen Probe korreliert sein kann.

10. Verfahren zum Nachweis jedes PTLV-Stamms nach Anspruch 9, **dadurch gekennzeichnet, dass** der Schritt der Amplifikation das Durchführen von zwei Amplifikationsreaktionen umfasst, wobei die zweite Reaktion auf einer Probe von Produkten ausgeführt wird, die im Rahmen der ersten Reaktion erhalten wurden, mit Hilfe der gleichen 5'-Oligonukleotide wie im Fall der ersten Reaktion, und der Oligonukleotide 3', die von jenen verschieden sind, die in der ersten Reaktion verwendet wurden, nämlich so genannter "nested" 3'-Primer, die mit einer Region hybridisieren, die weiter stromaufwärts der Sequenz liegt, die für die SU kodiert als die 3'-Primer, die in der ersten Reaktion verwendet werden.

11. Verfahren zum Nachweis jedes PTLV-Stamms nach Anspruch 9 oder 10, **dadurch gekennzeichnet, dass** es umfasst:
- eine erste Genamplifikationsreaktion, die mit Hilfe von Paaren degenerierter Oligonukleotide ausgeführt wird, ausgewählt aus den Paaren:
* Oligonukleotide mit SEQ ID Nr. 5/Oligonukleotide mit SEQ ID Nr. 18, oder
* Oligonukleotide mit SEQ ID Nr. 5/Oligonukleotide mit SEQ ID Nr. 27, oder
* Oligonukleotide mit SEQ ID Nr. 8/Oligonukleotide mit SEQ ID Nr. 27,
- einen zweiten Schritt der Amplifikation der Kopienanzahl der DNA-Fragmente, die im vorhergehenden Schritt erhalten wurden, mit Hilfe von Paaren degenerierter Oligonukleotide, jeweils ausgewählt aus den Paaren:
* Oligonukleotide mit SEQ ID Nr. 5/Oligonukleotide mit SEQ ID Nr. 13, oder
* Oligonukleotide mit SEQ ID Nr. 5/Oligonukleotide mit SEQ ID Nr. 13, oder Oligonukleotide mit SEQ ID Nr. 18, oder
* Oligonukleotide mit SEQ ID Nr. 8/Oligonukleotide mit SEQ ID Nr. 18,
- der Nachweis von DNA-Fragmenten, die im vorhergehenden Schritt amplifiziert wurden, wobei dieser Nachweis mit dem Nachweis, und gegebenenfalls der Identifikation von PTLV in der biologischen Probe korreliert sein kann.

12. Verfahren zum Nachweis jedes PTLV-Stamms nach einem der Ansprüche 9 bis 11, **dadurch gekennzeichnet, dass** es umfasst:
- eine erste Genamplifikationsreaktion, die mit Hilfe von Paaren degenerierter Oligonukleotide ausgeführt wird, die derart ausgewählt sind, dass:
* die degenerierten 5'-Oligonukleotide Folgende sind:
| | | |
|---|---|---|
| PTLVE5'83b | TAYBTNTTYCCNCATTGG | SEQ ID Nr. 5 |
wobei Y, B und N sind, wie in Anspruch 2 definiert,
* die degenerierten 3'-Oligonukleotide Folgende sind:
| | | |
|---|---|---|
| PTLVE3'241b | RTANARNACGTGCCA | SEQ ID Nr. 29 |
wobei R und N sind, wie in Anspruch 3 definiert,
- eine zweite Genamplifikationsreaktion, die mit Hilfe von Paaren degenerierter Oligonukleotide ausgeführt wird, die derart ausgewählt sind, dass:
* die degenerierten Oligonukleotide 5' Folgende sind:
| | | |
|---|---|---|
| PTLVE5'83b | TAYBTNTTYCCNCATTGG | SEQ ID Nr. 5 |
wobei Y, B und N sind, wie in Anspruch 2 definiert,
* die degenerierten Oligonukleotide 3' Folgende sind:
| | | |
|---|---|---|
| PTLVE3'145a | NACYTCYTGNGTAAAATT | SEQ ID Nr. 14, |
wobei Y und N sind, wie in Anspruch 3 definiert,

13. Materialsatz oder Kit zum Durchführen eines Verfahrens zum Nachweis nach einem der Ansprüche 9 bis 12, **dadurch gekennzeichnet, dass** es ein Primerpaar, wie in einem der Ansprüche 1 bis 7 definiert, und gegebenenfalls die Reagenzien umfasst, die für das Durchführen der PCR-Amplifikationsreaktion und für den Nachweis der amplifizierten Fragmente erforderlich sind.

14. Anwendung des Verfahrens zum Nachweis nach einem der Ansprüche 9 bis 12:
- zur In-vitro-Diagnose von Pathologien, die mit einer Infektion durch einen PTLV, oder durch einen Virus, der Sequenzen umfasst, die mit den SU der PTLV verwandt sind, beim Menschen oder beim Tier, verbunden sind, wie etwa Hämopathien, Autoimmunerkrankungen, entzündliche Erkrankungen, degenerative Erkrankungen,
- zum Screenen und zur Identifikation neuer Infektionserreger beim Menschen oder beim Tier, und insbesondere neuer Stämme oder Varianten von Viren, die den PTLV zugeordnet werden können, oder von Viren, die Sequenzen umfassen, die mit den SU der PTLV verwandt sind,
- zum Screenen von Prädispositions- oder Resistenzgenen gegenüber Pathologien beim Menschen oder beim Tier, die mit der Gegenwart der PTLV oder verwandter Sequenzen oder mit einer Infektion durch einen PTLV verbunden sind, wie etwa Hämopathien, Autoimmunerkrankungen, degenerative Erkrankungen,
- zum Screenen oder zum Design neuer Therapeutika, die vollständige oder partielle Sequenzen der Hüllproteine neuer, auf diese Weise nachgewiesener PTLV-Varianten umfassen,
- zum Screenen oder zum Design neuer Vektoren zur Zelltherapie, die die Tropismuseigenschaften der vollständigen oder partiellen Sequenzen der Hüllproteine neuer, auf diese Weise nachgewiesener PTLV-Varianten verwenden.


### LISTE DE SEQUENCES

<110> CENTRE NATIONAL DE LA RECHERCHE SCIENTIFIQUE
<120> OLIGONUCLEOTIDES ISSUS DES SEQUENCES CODANT POUR LA COMPOSANTE DE SURFACE DES PROTEINES D'ENVELOPPE DES PTLV ET LEURS UTILISATIONS
<130> IFB 02 BC CNR PTLV
<140> FR 02/05001
   <141> 2002-04-22
<160> 47
<170> PatentIn version 3.1
<210> 1
   <211> 6
   <212> PRT
   <213> Human T-cell lymphotropic virus type 1
<220>
   <221> misc_feature
   <222> (2)..(2)
   <223> L ou V
<400> 1
   Tyr Xaa Phe Pro His Trp
   1               5
<210> 2
   <211> 6
   <212> PRT
   <213> Human T-cell lymphotropic virus type 1
<220>
   <221> misc_feature
   <222> (4).. (4)
   <223> Q ou R
<400> 2
   Asn Phe Thr Xaa Glu Val
   1               5
<210> 3
   <211> 7
   <212> PRT
   <213> Human T-cell lymphotropic virus type 1
<220>
   <221> misc_feature
   <222> (3)..(3)
   <223> S ou T
<220>
   <221> misc_feature
   <222> (5)..(5)
   <223> I ou M
<400> 3
   Asn Tyr Xaa Cys Xaa Val Cys
   1               5
<210> 4
   <211> 5
   <212> PRT
   <213> Human T-cell lymphotropic virus type 1
<400> 4
   Trp His Val Leu Tyr
   1               5
<210> 5
   <211> 18
   <212> ADN
   <213> séquence artificielle
<220>
   <223> oligonucléotide 5' issu du brin ADN (+) correspondant au fragment polypeptidique 83-88 de la protéine d'enveloppe de la souche MT-2 de HTLV-1
<220>
   <221> misc_feature
   <222> (3).. (3)
   <223> C ou T
<220>
   <221> misc_feature
   <222> (4).. (4)
   <223> C, G ou T
<220>
   <221> misc_feature
   <222> (6).. (6)
   <223> A, C, G ou T
<220>
   <221> misc_feature
   <222> (9)..(9)
   <223> C ou T
<220>
   <221> misc_feature
   <222> (12)..(12)
   <223> A, C, G ou T
<220>
   <221> misc_feature
   <222> (15)..(15)
   <223> C ou T
<400> 5
   tanntnttnc cncantgg
<210> 6
   <211> 18
   <212> ADN
   <213> séquence artificielle
<220>
   <223> oligonucléotide 5' issu du brin ADN (+) correspondant au fragment polypeptidique 83-88 de la protéine d'enveloppe de la souche MT-2 de HTLV-1
<220>
   <221> misc_feature
   <222> (3)..(3)
   <223> C ou T
<220>
   <221> misc_feature
   <222> (4).. (4)
   <223> C, G ou T
<220>
   <221> misc_feature
   <222> (6)..(6)
   <223> A, C, G ou T
<220>
   <221> misc_feature
   <222> (9)..(9)
   <223> C ou T
<220>
   <221> misc_feature
   <222> (12)..(12)
   <223> A, C, G ou T
<400> 6
   tanntnttnc cncactgg
<210> 7
   <211> 18
   <212> ADN
   <213> séquence artificielle
<220>
   <223> oligonucléotide 5' issu du brin ADN (+) correspondant au fragment polypeptidique 83-88 de la protéine d'enveloppe de la souche MT-2 de HTLV-1
<220>
   <221> misc_feature
   <222> (3).. (3)
   <223> C ou T
<220>
   <221> misc_feature
   <222> (4).. (4)
   <223> C, G ou T
<220>
   <221> misc_feature
   <222> (6).. (6)
   <223> A, C, G ou T
<220>
   <221> misc_feature
   <222> (9) .. (9)
   <223> C ou T
<220>
   <221> misc_feature
   <222> (12) (12)
   <223> A, C, G ou T
<400> 7
   tanntnttnc cncattgg 18
<210> 8
   <211> 17
   <212> ADN
   <213> séquence artificielle
<220>
   <223> oligonucléotide 5' issu du brin ADN (+) correspondant au fragment polypeptidique 140-145 de la protéine d'enveloppe de la souche MT-2 de HTLV-1
<220>
   <221> misc_feature
   <222> (3)..(3)
   <223> C ou T
<220>
   <221> misc_feature
   <222> (6)..(6)
   <223> C ou T
<220>
   <221> misc_feature
   <222> (9)..(9)
   <223> A, C, G ou T
<220>
   <221> misc_feature
   <222> (12)..(12)
   <223> A ou G
<220>
   <221> misc_feature
   <222> (15)..(15)
   <223> A ou G
<400> 8
   aanttnacnc angangt 17
<210> 9
   <211> 17
   <212> ADN
   <213> séquence artificielle
<220>
   <223> oligonucléotide 5' issu du brin ADN (+) correspondant au fragment polypeptidique 140-145 de la protéine d'enveloppe de la souche MT-2 de HTLV-1
<220>
   <221> misc_feature
   <222> (3).. (3)
   <223> C ou T
<220>
   <221> misc_feature
   <222> (6)..(6)
   <223> C ou T
<220>
   <221> misc_feature
   <222> (9)..(9)
   <223> A, C, G ou T
<400> 9
   aanttnacnc aagaagt 17
<210> 10
   <211> 17
   <212> ADN
   <213> séquence artificielle
<220>
   <223> oligonucléotide 5' issu du brin ADN (+) correspondant au fragment polypeptidique 140-145 de la protéine d'enveloppe de la souche MT-2 de HTLV-1
<220>
   <221> misc_feature
   <222> (3)..(3)
   <223> C ou T
<220>
   <221> misc_feature
   <222> (6)..(6)
   <223> C ou T
<220>
   <221> misc_feature
   <222> (9).. (9)
   <223> A, C, G ou T
<400> 10
   aanttnacnc aggaagt 17
<210> 11
   <211> 17
   <212> ADN
   <213> séquence artificielle
<220>
   <223> oligonucléotide 5' issu du brin ADN (+) correspondant au fragment polypeptidique 140-145 de la protéine d'enveloppe de la souche MT-2 de HTLV-1
<220>
   <221> misc_feature
   <222> (3) ..(3)
   <223> C ou T
<220>
   <221> misc_feature
   <222> (6).. (6)
   <223> C ou T
<220>
   <221> misc_feature
   <222> (9).. (9)
   <223> A, C, G ou T
<400> 11
   aanttnacnc aagaggt 17
<210> 12
   <211> 17
   <212> ADN
   <213> séquence artificielle
<220>
   <223> oligonucléotide 5' issu du brin ADN (+) correspondant au fragment polypeptidique 140-145 de la protéine d'enveloppe de la souche MT-2 de HTLV-1
<220>
   <221> misc_feature
   <222> (3) .. (3)
   <223> C ou T
<220>
   <221> misc_feature
   <222> (6)..(6)
   <223> C ou T
<220>
   <221> misc_feature
   <222> (9).. (9)
   <223> A, C, G ou T
<400> 12
   aanttnacnc aggaggt 17
<210> 13
   <211> 18
   <212> ADN
   <213> séquence artificielle
<220>
   <223> oligonucléotide 3' issu du brin ADN (-) correspondant au fragment polypeptidique 140-145 de la protéine d'enveloppe de la souche MT-2 de HTLV-1
<220>
   <221> misc_feature
   <222> (1)..(1)
   <223> A, C, G ou T
<220>
   <221> misc_feature
   <222> (4)..(4)
   <223> C ou T
<220>
   <221> misc_feature
   <222> (7)..(7)
   <223> C ou T
<220>
   <221> misc_feature
   <222> (10)..(10)
   <223> A, C, G ou T
<220>
   <221> misc_feature
   <222> (13)..(13)
   <223> A ou G
<220>
   <221> misc_feature
   <222> (16) . (16)
   <223> A ou G
<400> 13
   nacntcntgn gtnaantt 18
<210> 14
   <211> 18
   <212> ADN
   <213> séquence artificielle
<220>
   <223> oligonucléotide 3' issu du brin ADN (-) correspondant au fragment polypeptidique 140-145 de la protéine d'enveloppe de la souche MT-2 de HTLV-1
<220>
   <221> misc_feature
   <222> (1)..(1)
   <223> A, C, G ou T
<220>
   <221> misc_feature
   <222> (4)..(4)
   <223> C ou T
<220>
   <221> misc_feature
   <222> (7)..(7)
   <223> C ou T
<220>
   <221> misc_feature
   <222> (10)..(10)
   <223> A, C, G ou T
<400> 14
   nacntcntgn gtaaaatt 18
<210> 15
   <211> 18
   <212> ADN
   <213> séquence artificielle
<220>
   <223> oligonucléotide 3' issu du brin ADN (-) correspondant au fragment polypeptidique 140-145 de la protéine d'enveloppe de la souche MT-2 de HTLV-1
<220>
   <221> misc_feature
   <222> (1)..(1)
   <223> A, C, G ou T
<220>
   <221> misc_feature
   <222> (4)..(4)
   <223> C ou T
<220>
   <221> misc_feature
   <222> (7).. (7)
   <223> C ou T
<220>
   <221> misc_feature
   <222> (10)..(10)
   <223> A, C, G ou T
<400> 15
   nacntcntgn gtgaaatt 18
<210> 16
   <211> 18
   <212> ADN
   <213> séquence artificielle
<220>
   <223> oligonucléotide 3' issu du brin ADN (-) correspondant au fragment polypeptidique 140-145 de la protéine d'enveloppe de la souche MT-2 de HTLV-1
<220>
   <221> misc_feature
   <222> (1).. (1)
   <223> A, C, G ou T
<220>
   <221> misc_feature
   <222> (4)..(4)
   <223> C ou T
<220>
   <221> misc_feature
   <222> (7)..(7)
   <223> C ou T
<220>
   <221> misc_feature
   <222> (10)..(10)
   <223> A, C, G ou T
<400> 16
   nacntcntgn gtaaagtt 18
<210> 17
   <211> 18
   <212> ADN
   <213> séquence artificielle
<220>
   <223> oligonucléotide 3' issu du brin ADN (-) correspondant au fragment polypeptidique 140-145 de la protéine d'enveloppe de la souche MT-2 de HTLV-1
<220>
   <221> misc_feature
   <222> (1)..(1)
   <223> A, C, G ou T
<220>
   <221> misc_feature
   <222> (4)..(4)
   <223> C ou T
<220>
   <221> misc_feature
   <222> (7)..(7)
   <223> C ou T
<220>
   <221> misc_feature
   <222> (10)..(10)
   <223> A, C, G ou T
<400> 17
   nacntcntgn gtgaagtt 18
<210> 18
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1) .. (1)
   <223> A ou G
<220>
   <221> misc_feature
   <222> (2)..(2)
   <223> A ou C
<220>
   <221> misc_feature
   <222> (3)..(3)
   <223> A, C, G ou T
<220>
   <221> misc_feature
   <222> (6)..(6)
   <223> A, C, G ou T
<220>
   <221> misc_feature
   <222> (9)..(9)
   <223> A ou G
<220>
   <221> misc_feature
   <222> (12).. (12)
   <223> A, C, G ou T
<220>
   <221> misc_feature
   <222> (13)..(13)
   <223> C ou G
<220>
   <221> misc_feature
   <222> (14)..(14)
   <223> A ou T
<220>
   <221> misc_feature
   <222> (15)..(15)
   <223> A ou G
<220>
   <221> misc_feature
   <222> (18)..(18)
   <223> A ou G
<400> 18
   nnnacnatnc annwntantt 20
<210> 19
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1)..(1)
   <223> A ou G
<220>
   <221> misc_feature
   <222> (2)..(2)
   <223> A ou C
<220>
   <221> misc_feature
   <222> (3)..(3)
   <223> A, C, G ou T
<220>
   <221> misc_feature
   <222> (6)..(6)
   <223> A, C, G ou T
<220>
   <221> misc_feature
   <222> (9)..(9)
   <223> A ou G
<220>
   <221> misc_feature
   <222> (12)..(12)
   <223> A, C, G ou T
<220>
   <221> misc_feature
   <222> (13)..(13)
   <223> C ou G
<400> 19
   nnnacnatnc annaataatt 20
<210> 20
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1)..(1)
   <223> A ou G
<220>
   <221> misc_feature
   <222> (2)..(2)
   <223> A ou C
<220>
   <221> misc_feature
   <222> (3)..(3)
   <223> A, C, G ou T
<220>
   <221> misc_feature
   <222> (6)..(6)
   <223> A, C, G ou T
<220>
   <221> misc_feature
   <222> (9).. (9)
   <223> A ou G
<220>
   <221> misc_feature
   <222> (12)..(12)
   <223> A, C, G ou T
<220>
   <221> misc_feature
   <222> (13)..(13)
   <223> C ou G
<400> 20
   nnnacnatnc annagtaatt 20
<210> 21
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1)..(1)
   <223> A ou G
<220>
   <221> misc_feature
   <222> (2)..(2)
   <223> A ou C
<220>
   <221> misc_feature
   <222> (3)..(3)
   <223> A, C, G ou T
<220>
   <221> misc_feature
   <222> (6).. (6)
   <223> A, C, G ou T
<220>
   <221> misc_feature
   <222> (9)..(9)
   <223> A ou G
<220>
   <221> misc_feature
   <222> (12)..(12)
   <223> A, C, G ou T
<220>
   <221> misc_feature
   <222> (13)..(13)
   <223> C ou G
<400> 21
   nnnacnatnc annaatagtt 20
<210> 22
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1)..(1)
   <223> A ou G
<220>
   <221> misc_feature
   <222> (2)..(2)
   <223> A ou C
<220>
   <221> misc_feature
   <222> (3)..(3)
   <223> A, C, G ou T
<220>
   <221> misc_feature
   <222> (6)..(6)
   <223> A, C, G ou T
<220>
   <221> misc_feature
   <222> (9)..(9)
   <223> A ou G
<220>
   <221> misc_feature
   <222> (12)..(12)
   <223> A, C, G ou T
<220>
   <221> misc_feature
   <222> (13)..(13)
   <223> C ou G
<400> 22
   nnnacnatnc annagtagtt 20
<210> 23
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1).. (1)
   <223> A ou G
<220>
   <221> misc_feature
   <222> (2)..(2)
   <223> A ou C
<220>
   <221> misc_feature
   <222> (3)..(3)
   <223> A, C, G ou T
<220>
   <221> misc_feature
   <222> (6)..(6)
   <223> A, C, G ou T
<220>
   <221> misc_feature
   <222> (9)..(9)
   <223> A ou G
<220>
   <221> misc_feature
   <222> (12)..(12)
   <223> A, C, G ou T
<220>
   <221> misc_feature
   <222> (13)..(13)
   <223> C ou G
<400> 23
   nnnacnatnc anntataatt 20
<210> 24
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1) ..(1)
   <223> A ou G
<220>
   <221> misc_feature
   <222> (2).. (2)
   <223> A ou C
<220>
   <221> misc_feature
   <222> (3)..(3)
   <223> A, C, G ou T
<220>
   <221> misc_feature
   <222> (6).. (6)
   <223> A, C, G ou T
<220>
   <221> misc_feature
   <222> (9)..(9)
   <223> A ou G
<220>
   <221> misc_feature
   <222> (12)..(12)
   <223> A, C, G ou T
<220>
   <221> misc_feature
   <222> (13).. (13)
   <223> C ou G
<400> 24
   nnnacnatnc anntgtaatt 20
<210> 25
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1)..(1)
   <223> A ou G
<220>
   <221> misc_feature
   <222> (2)..(2)
   <223> A ou C
<220>
   <221> misc_feature
   <222> (3).. (3)
   <223> A, C, G ou T
<220>
   <221> misc_feature
   <222> (6)..(6)
   <223> A, C, G ou T
<220>
   <221> misc_feature
   <222> (9)..(9)
   <223> A ou G
<220>
   <221> misc_feature
   <222> (12)..(12)
   <223> A, C, G ou T
<220>
   <221> misc_feature
   <222> (13)..(13)
   <223> C ou G
<400> 25
   nnnacnatnc anntatagtt 20
<210> 26
   <211> 20
   <212> ADN
   <213> séquence artificielle
<220>
   <223> oligonucléotide 3' issu du brin ADN (-) correspondant au fragment polypeptidique 222-228 de la protéine d'enveloppe de la souche MT-2 de HTLV-1
<220>
   <221> misc_feature
   <222> (1)..(1)
   <223> A ou G
<220>
   <221> misc_feature
   <222> (2') ..(2)
   <223> A ou C
<220>
   <221> misc_feature
   <222> (3).. (3)
   <223> A, C, G ou T
<220>
   <221> misc_feature
   <222> (6)..(6)
   <223> A, C, G ou T
<220>
   <221> misc_feature
   <222> (9).. (9)
   <223> A ou G
<220>
   <221> misc_feature
   <222> (12)..(12)
   <223> A, C, G ou T
<220>
   <221> misc_feature
   <222> (13)..(13)
   <223> C ou G
<400> 26
   nnnacnatnc anntgtagtt 20
<210> 27
   <211> 15
   <212> ADN
   <213> séquence artificielle
<220>
   <223> oligonucléotide 3' issu du brin ADN (-) correspondant au fragment polypeptidique 237-241 de la protéine d'enveloppe de la souche MT-2 de HTLV-1
<220>
   <221> misc_feature
   <222> (1)..(1)
   <223> A ou G
<220>
   <221> misc_feature
   <222> (4)..(4)
   <223> A, C, G ou T
<220>
   <221> misc_feature
   <222> (6)..(6)
   <223> A ou G
<220>
   <221> misc_feature
   <222> (7)..(7)
   <223> A, C, G ou T
<220>
   <221> misc_feature
   <222> (10)..(10)
   <223> A ou G
<400> 27
   ntanannacn tgcca 15
<210> 28
   <211> 15
   <212> ADN
   <213> séquence artificielle
<220>
   <223> oligonucléotide 3' issu du brin ADN (-) correspondant au fragment polypeptidique 237-241 de la protéine d'enveloppe de la souche MT-2 de HTLV-1
<220>
   <221> misc_feature
   <222> (1)..(1)
   <223> A ou G
<220>
   <221> misc_feature
   <222> (4) .. (4)
   <223> A, C, G ou T
<220>
   <221> misc_feature
   <222> (6)..(6)
   <223> A ou G
<220>
   <221> misc_feature
   <222> (7)..(7)
   <223> A, C, G ou T
<400> 28
   ntanannaca tgcca 15
<210> 29
   <211> 15
   <212> ADN
   <213> séquence artificielles
<220>
   <223> oligonucléotide 3' issu du brin ADN (-) correspondant au fragment polypeptidique 237-241 de la protéine d'enveloppe de la souche MT-2 de HTLV-1
<220>
   <221> misc_feature
   <222> (1)..(1)
   <223> A ou G
<220>
   <221> misc_feature
   <222> (4)..(4)
   <223> A, C, G ou T
<220>
   <221> misc_feature
   <222> (6)..(6)
   <223> A ou G
<220>
   <221> misc_feature
   <222> (7).. (7)
   <223> A, C, G ou T
<400> 29
   ntanannacg tgcca 15
<210> 30
   <211> 153
   <212> ADN
   <213> Human T-cell lymphotropic virus type 1
<220>
   <221> CDS
   <222> (1)..(153)
   <223>
<400> 30
   att aaa aag cca aac cca aat ggc gga ggc tat tat tta gcc tct tat        48
   Ile Lys Lys Pro Asn Pro Asn Gly Gly Gly Tyr Tyr Leu Ala Ser Tyr
   1               5                   10                  15
   tca gac cct tgt tcc tta aaa tgc cca tac ctg ggg tgc caa tca tgg        96
   Ser Asp Pro Cys Ser Leu Lys Cys Pro Tyr Leu Gly Cys Gln Ser Trp
               20                  25                  30
   acc tgc ccc tat aca gga gcc gtc tcc agc ccc tac tgg aag ttt cag       144
   Thr Cys Pro Tyr Thr Gly Ala Val Ser Ser Pro Tyr Trp Lys Phe Gln
           35                  40                  45
   caa gat gtc                                                           153
   Gln Asp Val
       50
<210> 31
   <211> 51
   <212> PRT
   <213> Human T-cell lymphotropic virus type 1
<400> 31
   Ile Lys Lys Pro Asn Pro Asn Gly Gly Gly Tyr Tyr Leu Ala Ser Tyr
   1               5                   10                  15
   Ser Asp Pro Cys Ser Leu Lys Cys Pro Tyr Leu Gly Cys Gln Ser Trp
               20                  25                  30
   Thr Cys Pro Tyr Thr Gly Ala Val Ser Ser Pro Tyr Trp Lys Phe Gln
           35                  40                  45
   Gln Asp Val
       50
<210> 32
   <211> 153
   <212> ADN
   <213> Human T-cell lymphotropic virus type 1
<220>
   <221> CDS
   <222> (1) .. (153)
   <223>
<400> 32
   gtt aaa aag cca aac cga aat ggc gga ggc tat tat tta gcc tct tat        48
   Val Lys Lys Pro Asn Arg Asn Gly Gly Gly Tyr Tyr Leu Ala Ser Tyr
   1               5                   10                  15
   tca gac cct tgt tcc tta aaa tgc cca tac ctg ggg tgc caa tca tgg        96
   Ser Asp Pro Cys Ser Leu Lys Cys Pro Tyr Leu Gly Cys Gln Ser Trp
               20                  25                  30
   acc tgc ccc tat aca gga gcc gtc tcc agc ccc tac tgg aag ttt cag       144
   Thr Cys Pro Tyr Thr Gly Ala Val Ser Ser Pro Tyr Trp Lys Phe Gln
           35                  40                  45
   caa gat gtc                                                           153
   Gln Asp Val
       50
<210> 33
   <211> 51
   <212> PRT
   <213> Human T-cell lymphotropic virus type 1
<400> 33
   Val Lys Lys Pro Asn Arg Asn Gly Gly Gly Tyr Tyr Leu Ala Ser Tyr
   1               5                   10                  15
   Ser Asp Pro Cys Ser Leu Lys Cys Pro Tyr Leu Gly Cys Gln Ser Trp
               20                  25                  30
   Thr Cys Pro Tyr Thr Gly Ala Val Ser Ser Pro Tyr Trp Lys Phe Gln
           35                  40                  45
   Gln Asp Val
       50
<210> 34
   <211> 153
   <212> ADN
   <213> Human T-cell lymphotropic virus type 1
<220>
   <221> CDS
   <222> (1) .. (153)
   <223>
<400> 34
   att aaa aag cca aac cga aat ggc gga ggc tat tat tta gcc tct tat      48
   Ile Lys Lys Pro Asn Arg Asn Gly Gly Gly Tyr Tyr Leu Ala Ser Tyr
   1               5                   10                  15
   tca gac cct tgt tcc tta aaa tgc cca tac ctg ggg tgc caa tca tgg      96
   Ser Asp Pro Cys Ser Leu Lys Cys Pro Tyr Leu Gly Cys Gln Ser Trp
               20                  25                  30
   acc tgc ccc tat aca gga gcc gtc tcc agc ccc tac tgg aag ttt caa     144
   Thr Cys Pro Tyr Thr Gly Ala Val Ser Ser Pro Tyr Trp Lys Phe Gln
           35                  40                  45
   caa gat gtc                                                         153
   Gln Asp Val
       50
<210> 35
   <211> 51
   <212> PRT
   <213> Human T-cell lymphotropic virus type 1
<400> 35
   Ile Lys Lys Pro Asn Arg Asn Gly Gly Gly Tyr Tyr Leu Ala Ser Tyr
   1               5                   10                  15
   Ser Asp Pro Cys Ser Leu Lys Cys Pro Tyr Leu Gly Cys Gln Ser Trp
               20                  25                  30
   Thr Cys Pro Tyr Thr Gly Ala Val Ser Ser Pro Tyr Trp Lys Phe Gln
           35                  40                  45
   Gln Asp Val
       50
<210> 36
   <211> 153
   <212> ADN
   <213> Human T-cell lymphotropic virus type 1
<220>
   <221> CDS
   <222> (1)..(153)
   <223>
<400> 36
   gtt aaa aag cca aac cga aat ggc gga ggc tat tat tta gcc tct tat        48
   Val Lys Lys Pro Asn Arg Asn Gly Gly Gly Tyr Tyr Leu Ala Ser Tyr
   1               5                   10                  15
   tca gac cct tgt tcc tta aaa tgc cca tac ctg ggg tgc caa tca tgg        96
   Ser Asp Pro Cys Ser Leu Lys Cys Pro Tyr Leu Gly Cys Gln Ser Trp
               20                  25                  30
   acc tgc ccc tat aca gga ccc gtc tcc agc ccc tac tgg aag ttt cag       144
   Thr Cys Pro Tyr Thr Gly Pro Val Ser Ser Pro Tyr Trp Lys Phe Gln
           35                  40                  45
   caa gat gtc                                                           153
   Gln Asp Val
       50
<210> 37
   <211> 51
   <212> PRT
   <213> Human T-cell lymphotropic virus type 1
<400> 37
   Val Lys Lys Pro Asn Arg Asn Gly Gly Gly Tyr Tyr Leu Ala Ser Tyr
   1               5                   10                  15
   Ser Asp Pro Cys Ser Leu Lys Cys Pro Tyr Leu Gly Cys Gln Ser Trp
               20                  25                  30
   Thr Cys Pro Tyr Thr Gly Pro Val Ser Ser Pro Tyr Trp Lys Phe Gln
           35                  40                  45
   Gln Asp Val
       50
<210> 38
   <211> 171
   <212> ADN
   <213> Human T-cell lymphotropic virus type 1
<220>
   <221> CDS
   <222> (1) .. (171)
   <223>
<400> 38
   att aaa aag cca aac cga aat ggc gga ggc tat cat tca gcc tct tat      48
   Ile Lys Lys Pro Asn Arg Asn Gly Gly Gly Tyr His Ser Ala Ser Tyr
   1               5                   10                  15
   tca gac cct tgt tcc tta aag tgc cca tac ctg ggg tgc caa tca tgg      96
   Ser Asp Pro Cys Ser Leu Lys Cys Pro Tyr Leu Gly Cys Gln Ser Trp
               20                  25                  30
   acc tgc ccc tat gca gga gcc gtc tcc agc ccc tac tgg aag ttt cag     144
   Thr Cys Pro Tyr Ala Gly Ala Val Ser Ser Pro Tyr Trp Lys Phe Gln
           35                  40                  45
   caa gat gtc aat ttt acc cag gaa gta                                 171
   Gln Asp Val Asn Phe Thr Gln Glu Val
       50                  55
<210> 39
   <211> 57
   <212> PRT
   <213> Human T-cell lymphotropic virus type 1
<400> 39
   Ile Lys Lys Pro Asn Arg Asn Gly Gly Gly Tyr His Ser Ala Ser Tyr
   1               5                   10                  15
   Ser Asp Pro Cys Ser Leu Lys Cys Pro Tyr Leu Gly Cys Gln Ser Trp
               20                  25                  30
   Thr Cys Pro Tyr Ala Gly Ala Val Ser Ser Pro Tyr Trp Lys Phe Gln
           35                  40                  45
   Gln Asp Val Asn Phe Thr Gln Glu Val
       50                  55
<210> 40
   <211> 153
   <212> ADN
   <213> Human T-cell lymphotropic virus type 2
<220>
   <221> CDS
   <222> (1) .. (153)
   <223>
<400> 40
   ata aga aag cca aac aga cag ggc cta ggg tac tac tcg cct tcc tac       48
   Ile Arg Lys Pro Asn Arg Gln Gly Leu Gly Tyr Tyr Ser Pro Ser Tyr
   1               5                   10                  15
   aat gac cct tgc tcg cta caa tgc ccc tac ttg ggc tcc caa tca tgg       96
   Asn Asp Pro Cys Ser Leu Gln Cys Pro Tyr Leu Gly Ser Gln Ser Trp
               20                  25                  30
   aca tgc cca tac acg gcc ccc gtc tcc act cca tcc tgg aat ttt cat      144
   Thr Cys Pro Tyr Thr Ala Pro Val Ser Thr Pro Ser Trp Asn Phe His
           35                  40                  45
   tca gat gta                                                           153
   Ser Asp Val
       50
<210> 41
   <211> 51
   <212> PRT
   <213> Human T-cell lymphotropic virus type 2
<400> 41
   Ile Arg Lys Pro Asn Arg Gln Gly Leu Gly Tyr Tyr Ser Pro Ser Tyr
   1               5                   10                  15
   Asn Asp Pro Cys Ser Leu Gln Cys Pro Tyr Leu Gly Ser Gln Ser Trp
               20                  25                  30
   Thr Cys Pro Tyr Thr Ala Pro Val Ser Thr Pro Ser Trp Asn Phe His
           35                  40                  45
   Ser Asp Val
       50
<210> 42
   <211> 924
   <212> ADN
   <213> Human T-cell lymphotropic virus type 1
<220>
   <221> CDS
   <222> (1)..(924)
   <223>
<400> 42
   atg ggt aag ttt ctc gcc act ttg att tta ttc ttc cag ttc tgc ccc       48
   Met Gly Lys Phe Leu Ala Thr Leu Ile Leu Phe Phe Gln Phe Cys Pro
   1               5                   10                  15
   ctc atc ctc ggt gat tac agc ccc agc tgc tgt act ctc aca att gga       96
   Leu Ile Leu Gly Asp Tyr Ser Pro Ser Cys Cys Thr Leu Thr Ile Gly
               20                  25                  30
   gtc tcc tca tac cac tct aaa ccc tgc aat cct gcc cag cca gtt tgt      144
   Val Ser Ser Tyr His Ser Lys Pro Cys Asn Pro Ala Gln Pro Val Cys
           35                  40                  45
   tcg tgg acc ctc gac ctg ctg gcc ctt tca gcg gat cag gcc cta cag      192
   Ser Trp Thr Leu Asp Leu Leu Ala Leu Ser Ala Asp Gln Ala Leu Gln
       50                  55                  60
   ccc ccc tgc cct aat cta gta agt tac tcc agc tac cat gcc acc tat      240
   Pro Pro Cys Pro Asn Leu Val Ser Tyr Ser Ser Tyr His Ala Thr Tyr
   65                  70                  75                  80
   tcc cta tat cta ttc cct cat tgg att aaa aag cca aac cga aat ggc      288
   Ser Leu Tyr Leu Phe Pro His Trp Ile Lys Lys Pro Asn Arg Asn Gly
                   85                  90                  95
   gga ggc tat tat tca gcc tct tat tca gac cct tgt tcc tta aag tgc      336
   Gly Gly Tyr Tyr Ser Ala Ser Tyr Ser Asp Pro Cys Ser Leu Lys Cys
               100                 105                 110
   cca tac ctg ggg tgc caa tca tgg acc tgc ccc tat aca gga gcc gtc      384
   Pro Tyr Leu Gly Cys Gln Ser Trp Thr Cys Pro Tyr Thr Gly Ala Val
           115                 120                 125
   tcc agc ccc tac tgg aag ttt cag caa gat gtc aat ttt act caa gaa      432
   Ser Ser Pro Tyr Trp Lys Phe Gln Gln Asp Val Asn Phe Thr Gln Glu
       130                 135                 140
   gtt tca cgc ctc aat att aat ctc cat ttt tca aaa tgc ggt ttt ccc      480
   Val Ser Arg Leu Asn Ile Asn Leu His Phe Ser Lys Cys Gly Phe Pro
   145                 150                 155                 160
   ttc tcc ctt cta gtc gac gct cca gga tat gac ccc atc tgg ttc ctt      528
   Phe Ser Leu Leu Val Asp Ala Pro Gly Tyr Asp Pro Ile Trp Phe Leu
                   165                 170                 175
   aat acc gaa ccc agc caa ctg cct ccc acc gcc cct cct cta ctc ccc      576
   Asn Thr Glu Pro Ser Gln Leu Pro Pro Thr Ala Pro Pro Leu Leu Pro
               180                 185                 190
   cac tct aac cta gac cac atc ctc gag ccc tct ata cca tgg aaa tca      624
   His Ser Asn Leu Asp His Ile Leu Glu Pro Ser Ile Pro Trp Lys Ser
           195                 200                 205
   aaa ctc ctg acc ctt gtc cag tta acc cta caa agc act aat tat act      672
   Lys Leu Leu Thr Leu Val Gln Leu Thr Leu Gln Ser Thr Asn Tyr Thr
       210                 215                 220
   tgc att gtc tgt atc gat cgt gcc agc cta tcc act tgg cac gtc cta      720
   Cys Ile Val Cys Ile Asp Arg Ala Ser Leu Ser Thr Trp His Val Leu
   225                 230                 235                 240
   tac tct ccc aac gtc tct gtt cca tcc tct tct tct acc ccc ctc ctt      768
   Tyr Ser Pro Asn Val Ser Val Pro Ser Ser Ser Ser Thr Pro Leu Leu
                   245                 250                 255
   tac cca tcg tta gcg ctt cca gcc ccc cac ctg acg tta cca ttt aac      816
   Tyr Pro Ser Leu Ala Leu Pro Ala Pro His Leu Thr Leu Pro Phe Asn
               260                 265                 270
   tgg acc cac tgc ttt gac ccc cag att caa gct ata gtc tcc tcc ccc      864
   Trp Thr His Cys Phe Asp Pro Gln Ile Gln Ala Ile Val Ser Ser Pro
           275                 280                 285
   tgt cat aac tcc ctc atc ctg ccc ccc ttt tcc ttg tca cct gtt ccc      912
   Cys His Asn Ser Leu Ile Leu Pro Pro Phe Ser Leu Ser Pro Val Pro
       290                 295                 300
   acc cta gga tcc                                                      924
   Thr Leu Gly Ser
   305
<210> 43
   <211> 308
   <212> PRT
   <213> Human T-cell lymphotropic virus type 1
<400> 43
   Met Gly Lys Phe Leu Ala Thr Leu Ile Leu Phe Phe Gln Phe Cys Pro
   1               5                   10                  15
   Leu Ile Leu Gly Asp Tyr Ser Pro Ser Cys Cys Thr Leu Thr Ile Gly
               20                  25                  30
   Val Ser Ser Tyr His Ser Lys Pro Cys Asn Pro Ala Gln Pro Val Cys
           35                  40                  45
   Ser Trp Thr Leu Asp Leu Leu Ala Leu Ser Ala Asp Gln Ala Leu Gln
       50                  55                  60
   Pro Pro Cys Pro Asn Leu Val Ser Tyr Ser Ser Tyr His Ala Thr Tyr
   65                  70                  75                  80
   Ser Leu Tyr Leu Phe Pro His Trp Ile Lys Lys Pro Asn Arg Asn Gly
                   85                  90                  95
   Gly Gly Tyr Tyr Ser Ala Ser Tyr Ser Asp Pro Cys Ser Leu Lys Cys
               100                 105                 110
   Pro Tyr Leu Gly Cys Gln Ser Trp Thr Cys Pro Tyr Thr Gly Ala Val
           115                 120                 125
   Ser Ser Pro Tyr Trp Lys Phe Gln Gln Asp Val Asn Phe Thr Gln Glu
       130                 135                 140
   Val Ser Arg Leu Asn Ile Asn Leu His Phe Ser Lys Cys Gly Phe Pro
   145                 150                 155                 160
   Phe Ser Leu Leu Val Asp Ala Pro Gly Tyr Asp Pro Ile Trp Phe Leu
                   165                 170                 175
   Asn Thr Glu Pro Ser Gln Leu Pro Pro Thr Ala Pro Pro Leu Leu Pro
               180                 185                 190
   His Ser Asn Leu Asp His Ile Leu Glu Pro Ser Ile Pro Trp Lys Ser
           195                 200                 205
   Lys Leu Leu Thr Leu Val Gln Leu Thr Leu Gln Ser Thr Asn Tyr Thr
       210                 215                 220
   Cys Ile Val Cys Ile Asp Arg Ala Ser Leu Ser Thr Trp His Val Leu
   225                 230                 235                 240
   Tyr Ser Pro Asn Val Ser Val Pro Ser Ser Ser Ser Thr Pro Leu Leu
                   245                 250                 255
   Tyr Pro Ser Leu Ala Leu Pro Ala Pro His Leu Thr Leu Pro Phe Asn
               260                 265                 270
   Trp Thr His Cys Phe Asp Pro Gln Ile Gln Ala Ile Val Ser Ser Pro
           275                 280                 285
   Cys His Asn Ser Leu Ile Leu Pro Pro Phe Ser Leu Ser Pro Val Pro
       290                 295                 300
   Thr Leu Gly Ser
   305
<210> 44
   <211> 912
   <212> ADN
   <213> Human T-cell lymphotropic virus type 2
<220>
   <221> CDS
   <222> (1)..(912)
   <223>
<400> 44
   atg ggt aac gtt ttc ttc cta ctt tta ttc agt ctc aca cac ttc cca        48
   Met Gly Asn Val Phe Phe Leu Leu Leu Phe Ser Leu Thr His Phe Pro
   1               5                   10                  15
   cca gtc cag cag agc cga tgc aca ctc acg gtt ggt att tcc tcc tac        96
   Pro Val Gln Gln Ser Arg Cys Thr Leu Thr Val Gly Ile Ser Ser Tyr
               20                  25                  30
   cac tcc agc ccc tgt agc cca acc caa ccc gtc tgc acg tgg aac ctc       144
   His Ser Ser Pro Cys Ser Pro Thr Gln Pro Val Cys Thr Trp Asn Leu
           35                  40                  45
   gac ctt aat tcc cta acg acg gac cag cga cta cat ccc ccc tgc cct      192
   Asp Leu Asn Ser Leu Thr Thr Asp Gln Arg Leu His Pro Pro Cys Pro
       50                  55                  60
   aac cta att act tac tct ggc ttc cac aaa act tat tcc tta tac tta      240
   Asn Leu Ile Thr Tyr Ser Gly Phe His Lys Thr Tyr Ser Leu Tyr Leu
   65                  70                  75                  80
   ttc cca cat tgg ata aag aag cca aat aga cag ggc cta gga tac tac      288
   Phe Pro His Trp Ile Lys Lys Pro Asn Arg Gln Gly Leu Gly Tyr Tyr
                   85                  90                  95
   tcg ccc tcc tat aat gac cct tgc tcg cta caa tgc ccc tac tta ggc      336
   Ser Pro Ser Tyr Asn Asp Pro Cys Ser Leu Gln Cys Pro Tyr Leu Gly
               100                 105                 110
   tgc caa tca tgg aca tgc cca tac acg ggc ccc gtc tcc agt cca tcc      384
   Cys Gln Ser Trp Thr Cys Pro Tyr Thr Gly Pro Val Ser Ser Pro Ser
           115                 120                 125
   tgg aag ttt cac tca gat gta aat ttc acc caa gaa gtc agc caa gtg      432
   Trp Lys Phe His Ser Asp Val Asn Phe Thr Gln Glu Val Ser Gln Val
       130                 135                 140
   tcc ctt cga cta cac ttc tct aag tgc ggc tcc tcc atg acc ctt cta      480
   Ser Leu Arg Leu His Phe Ser Lys Cys Gly Ser Ser Met Thr Leu Leu
   145                 150                 155                 160
   gta gat gcc cct gga tat gat cct tta tgg ttc atc acc tca gaa ccc      528
   Val Asp Ala Pro Gly Tyr Asp Pro Leu Trp Phe Ile Thr Ser Glu Pro
                   165                 170                 175
   act cag cct ccc cca act cct ccc cca ctg gtc cat gac tcc gac ctt      576
   Thr Gln Pro Pro Pro Thr Pro Pro Pro Leu Val His Asp Ser Asp Leu
               180                 185                 190
   gaa cac gtc cta acc ccc tcc acg tct tgg aca acc aaa atg ctc aag      624
   Glu His Val Leu Thr Pro Ser Thr Ser Trp Thr Thr Lys Met Leu Lys
           195                 200                 205
   ttt atc cag ctg acc ttg cag agc acc aat tac tcc tgc atg gtt tgc      672
   Phe Ile Gln Leu Thr Leu Gln Ser Thr Asn Tyr Ser Cys Met Val Cys
       210                 215                 220
   gtg gat aga tcc agc ctc tca tcc tgg cat gtg ctc tac acc ccc aac      720
   Val Asp Arg Ser Ser Leu Ser Ser Trp His Val Leu Tyr Thr Pro Asn
   225                 230                 235                 240
   atc tcc att ccc caa caa acc tcc tcc cga acc atc ctc ttt cct tct      768
   Ile Ser Ile Pro Gln Gln Thr Ser Ser Arg Thr Ile Leu Phe Pro Ser
                   245                 250                 255
   ctt gcc ctg ccc gct cct cca ttc caa ccc ttc cct tgg acc cat tgc      816
   Leu Ala Leu Pro Ala Pro Pro Phe Gln Pro Phe Pro Trp Thr His Cys
               260                 265                 270
   tac caa cct cgc cta cag gca ata acg aca gat gac tgc aac aac tcc      864
   Tyr Gln Pro Arg Leu Gln Ala Ile Thr Thr Asp Asp Cys Asn Asn Ser
           275                 280                 285
   att atc ctc ccc cct ttt tcc ctc gcc ccc gta cct cct ccg gcg aca      912
   Ile Ile Leu Pro Pro Phe Ser Leu Ala Pro Val Pro Pro Pro Ala Thr
   290 295 300
<210> 45
   <211> 304
   <212> PRT
   <213> Human T-cell lymphotropic virus type 2
<400> 45
   Met Gly Asn Val Phe Phe Leu Leu Leu Phe Ser Leu Thr His Phe Pro
   1               5                   10                  15
   Pro Val Gln Gln Ser Arg Cys Thr Leu Thr Val Gly Ile Ser Ser Tyr
               20                  25                  30
   His Ser Ser Pro Cys Ser Pro Thr Gln Pro Val Cys Thr Trp Asn Leu
           35                  40                  45
   Asp Leu Asn Ser Leu Thr Thr Asp Gln Arg Leu His Pro Pro Cys Pro
       50                  55                  60
   Asn Leu Ile Thr Tyr Ser Gly Phe His Lys Thr Tyr Ser Leu Tyr Leu
   65                  70                  75                  80
   Phe Pro His Trp Ile Lys Lys Pro Asn Arg Gln Gly Leu Gly Tyr Tyr
                   85                  90                  95
   Ser Pro Ser Tyr Asn Asp Pro Cys Ser Leu Gln Cys Pro Tyr Leu Gly
               100                 105                 110
   Cys Gln Ser Trp Thr Cys Pro Tyr Thr Gly Pro Val Ser Ser Pro Ser
           115                 120                 125
   Trp Lys Phe His Ser Asp Val Asn Phe Thr Gln Glu Val Ser Gln Val
       130                 135                 140
   Ser Leu Arg Leu His Phe Ser Lys Cys Gly Ser Ser Met Thr Leu Leu
   145                 150                 155                 160
   Val Asp Ala Pro Gly Tyr Asp Pro Leu Trp Phe Ile Thr Ser Glu Pro
                   165                 170                 175
   Thr Gln Pro Pro Pro Thr Pro Pro Pro Leu Val His Asp Ser Asp Leu
               180                 185                 190
   Glu His Val Leu Thr Pro Ser Thr Ser Trp Thr Thr Lys Met Leu Lys
           195                 200                 205
   Phe Ile Gln Leu Thr Leu Gln Ser Thr Asn Tyr Ser Cys Met Val Cys
       210                 215                 220
   Val Asp Arg Ser Ser Leu Ser Ser Trp His Val Leu Tyr Thr Pro Asn
   225                 230                 235                 240
   Ile Ser Ile Pro Gln Gln Thr Ser Ser Arg Thr Ile Leu Phe Pro Ser
                   245                 250                 255
   Leu Ala Leu Pro Ala Pro Pro Phe Gln Pro Phe Pro Trp Thr His Cys
               260                 265                 270
   Tyr Gln Pro Arg Leu Gln Ala Ile Thr Thr Asp Asp Cys Asn Asn Ser
           275                 280                 285
   Ile Ile Leu Pro Pro Phe Ser Leu Ala Pro Val Pro Pro Pro Ala Thr
       290                 295                 300
<210> 46
   <211> 930
   <212> ADN
   <213> Simian T-cell lymphotropic virus type 3
<220>
   <221> CDS
   <222> (1)..(930)
   <223>
<400> 46
   atg ggt aag ttt ggc ctt tat tgt ctt gtt cac ctt tac ata ctt ctc         48
   Met Gly Lys Phe Gly Leu Tyr Cys Leu Val His Leu Tyr Ile Leu Leu
   1               5                   10                  15
   cct gcc tcc tct ggc aat ccc agt cgg tgc acc ctg ttc ata ggg gcc         96
   Pro Ala Ser Ser Gly Asn Pro Ser Arg Cys Thr Leu Phe Ile Gly Ala
               20                  25                  30
   tct tcc tac cac tcc agc cct tgc ggg tcc agc ctc cca cgg tgt acc        144
   Ser Ser Tyr His Ser Ser Pro Cys Gly Ser Ser Leu Pro Arg Cys Thr
           35                  40                  45
   tgg aat ctt gac cta ttc tcc ctc acg aaa gat caa agc cta agc ccc        192
   Trp Asn Leu Asp Leu Phe Ser Leu Thr Lys Asp Gln Ser Leu Ser Pro
       50                  55                  60
   cca tgt cca gac tta att act tac tca caa tac cac aag ccc tac tcc        240
   Pro Cys Pro Asp Leu Ile Thr Tyr Ser Gln Tyr His Lys Pro Tyr Ser
   65                  70                  75                  80
   ctg tat gta ttc cct cat tgg ata act aaa cct aac cgc cgg ggc tta        288
   Leu Tyr Val Phe Pro His Trp Ile Thr Lys Pro Asn Arg Arg Gly Leu
                   85                  90                  95
   ggt tac tat tcc gct tcc tac tca gac ccc tgt gcc ata cag tgc cct        336
   Gly Tyr Tyr Ser Ala Ser Tyr Ser Asp Pro Cys Ala Ile Gln Cys Pro
               100                 105                 110
   tac ctg gga tgc cag tcg tgg aca tgc ccc tat acg ggc ccg gtg tcc        384
   Tyr Leu Gly Cys Gln Ser Trp Thr Cys Pro Tyr Thr Gly Pro Val Ser
           115                 120                 125
   agt ccg cat tgg aga tac acc tat gat ctt aac ttt acc cag gag gta        432
   Ser Pro His Trp Arg Tyr Thr Tyr Asp Leu Asn Phe Thr Gln Glu Val
       130                 135                 140
   tca tcc gtc tcc tta cac ttg cat ttc tcc aaa tgc gga tcc tcg ttc        480
   Ser Ser Val Ser Leu His Leu His Phe Ser Lys Cys Gly Ser Ser Phe
   145                 150                 155                 160
   tcc ttt cta cta gac gca cca gga tat gac cca gtg tgg ttc ctc tcc        528
   Ser Phe Leu Leu Asp Ala Pro Gly Tyr Asp Pro Val Trp Phe Leu Ser
                   165                 170                 175
   tcc cag gcc aca cag gct cca ccc aca cct gcc cct ctc ata cgg gac        576
   Ser Gln Ala Thr Gln Ala Pro Pro Thr Pro Ala Pro Leu Ile Arg Asp
               180                 185                 190
   tca gat ctc cag tac att cta gaa ccg ccc att ccg tgg agc tct aag        624
   Ser Asp Leu Gln Tyr Ile Leu Glu Pro Pro Ile Pro Trp Ser Ser Lys
           195                 200                 205
   att ctt aac ctt atc ctc ctc acc cta aaa agc act aac tat tct tgc        672
   Ile Leu Asn Leu Ile Leu Leu Thr Leu Lys Ser Thr Asn Tyr Ser Cys
       210                 215                 220
   atg gtc tgt gtt gac cgc tcc agc cta tcc tca tgg cat gtc ctg tat        720
   Met Val Cys Val Asp Arg Ser Ser Leu Ser Ser Trp His Val Leu Tyr
   225                 230                 235                 240
   gga ccc act caa gtc ccc agt cca ccc gac ccc caa gcc cgg tct atc        768
   Gly Pro Thr Gln Val Pro Ser Pro Pro Asp Pro Gln Ala Arg Ser Ile
                   245                 250                 255
   ctg cga cct gcc tta gct att ccc gcc agt aat atc acc ccc ccg ttt        816
   Leu Arg Pro Ala Leu Ala Ile Pro Ala Ser Asn Ile Thr Pro Pro Phe
               260                 265                 270
   cct tgg acc cat tgc tat cgc cct cct ccg caa gcc atc tcc tcg gag        864
   Pro Trp Thr His Cys Tyr Arg Pro Pro Pro Gln Ala Ile Ser Ser Glu
           275                 280                 285
   aat tgt aac aac tct gta gtg ctg ccc ccc ttt tct ctg tct cca att        912
   Asn Cys Asn Asn Ser Val Val Leu Pro Pro Phe Ser Leu Ser Pro Ile
       290                 295                 300
   cct aac gtc tcc aga ccc                                                930
   Pro Asn Val Ser Arg Pro
   305                 310
<210> 47
   <211> 310
   <212> PRT
   <213> Simian T-cell lymphotropic virus type 3
<400> 47
   Met Gly Lys Phe Gly Leu Tyr Cys Leu Val His Leu Tyr Ile Leu Leu
   1               5                   10                  15
   Pro Ala Ser Ser Gly Asn Pro Ser Arg Cys Thr Leu Phe Ile Gly Ala
               20                  25                  30
   Ser Ser Tyr His Ser Ser Pro Cys Gly Ser Ser Leu Pro Arg Cys Thr
           35                  40                  45
   Trp Asn Leu Asp Leu Phe Ser Leu Thr Lys Asp Gln Ser Leu Ser Pro
       50                  55                  60
   Pro Cys Pro Asp Leu Ile Thr Tyr Ser Gln Tyr His Lys Pro Tyr Ser
   65                  70                  75                  80
   Leu Tyr Val Phe Pro His Trp Ile Thr Lys Pro Asn Arg Arg Gly Leu
                   85                  90                  95
   Gly Tyr Tyr Ser Ala Ser Tyr Ser Asp Pro Cys Ala Ile Gln Cys Pro
               100                 105                 110
   Tyr Leu Gly Cys Gln Ser Trp Thr Cys Pro Tyr Thr Gly Pro Val Ser
           115                 120                 125
   Ser Pro His Trp Arg Tyr Thr Tyr Asp Leu Asn Phe Thr Gln Glu Val
       130                 135                 140
   Ser Ser Val Ser Leu His Leu His Phe Ser Lys Cys Gly Ser Ser Phe
   145                 150                 155                 160
   Ser Phe Leu Leu Asp Ala Pro Gly Tyr Asp Pro Val Trp Phe Leu Ser
                   165                 170                 175
   Ser Gln Ala Thr Gln Ala Pro Pro Thr Pro Ala Pro Leu Ile Arg Asp
               180                 185                 190
   Ser Asp Leu Gln Tyr Ile Leu Glu Pro Pro Ile Pro Trp Ser Ser Lys
           195                 200                 205
   Ile Leu Asn Leu Ile Leu Leu Thr Leu Lys Ser Thr Asn Tyr Ser Cys
       210                 215                 220
   Met Val Cys Val Asp Arg Ser Ser Leu Ser Ser Trp His Val Leu Tyr
   225                 230                 235                 240
   Gly Pro Thr Gln Val Pro Ser Pro Pro Asp Pro Gln Ala Arg Ser Ile
                   245                 250                 255
   Leu Arg Pro Ala Leu Ala Ile Pro Ala Ser Asn Ile Thr Pro Pro Phe
               260                 265                 270
   Pro Trp Thr His Cys Tyr Arg Pro Pro Pro Gln Ala Ile Ser Ser Glu
           275                 280                 285
   Asn Cys Asn Asn Ser Val Val Leu Pro Pro Phe Ser Leu Ser Pro Ile
       290                 295                 300
   Pro Asn Val Ser Arg Pro
   305                 310